(19) Europäisches Patentamt
European Patent Office
Office européen des brevets

(11) EP 4 592 323 A1

(12) EUROPEAN PATENT APPLICATION
published in accordance with Art. 153(4) EPC

(43) Date of publication:
**30.07.2025 Bulletin 2025/31**

(21) Application number: **23868214.0**

(22) Date of filing: **20.09.2023**

(51) International Patent Classification (IPC):
***C08F 20/18*** (2006.01) ***C07C 67/03*** (2006.01)
***C07C 67/62*** (2006.01) ***C07C 69/54*** (2006.01)
***C07D 303/16*** (2006.01) ***C07D 307/12*** (2006.01)
***C08F 10/14*** (2006.01) ***C08F 20/20*** (2006.01)
***C08F 20/28*** (2006.01)

(52) Cooperative Patent Classification (CPC):
(C-Sets available)
**C07C 67/62; C07C 67/03; C07D 303/16; C07D 307/12; C08F 20/18; C08F 20/20; C08F 20/28** (Cont.)

(86) International application number:
**PCT/JP2023/034093**

(87) International publication number:
**WO 2024/063093 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.09.2022 JP 2022149219**
**20.09.2022 JP 2022149220**
**20.09.2022 JP 2022149221**

(71) Applicant: **Mitsubishi Chemical Corporation**
**Tokyo 100-8251 (JP)**

(72) Inventor: **WATANABE Takeshi**
**Tokyo 100-8251 (JP)**

(74) Representative: **Hoffmann Eitle**
**Patent- und Rechtsanwälte PartmbB**
**Arabellastraße 30**
**81925 München (DE)**

(54) **ESTER-COMPOUND-CONTAINING COMPOSITION AND METHOD FOR PRODUCING SAME, METHODS FOR PRODUCING POLYMERIZABLE COMPOSITION AND (METH)ACRYLIC POLYMER**

(57) An object of the present invention is to provide an ester-compound-containing composition having excellent storage stability and a method for producing the same, a polymerizable composition using the ester-compound-containing composition, a method for producing a (meth)acrylic polymer. The ester-compound-containing composition contains an ester compound (1) represented by Formula (1): $CH_2=CR^1-C(=O)-O-R^2$ (here, in Formula (1-1), $R^1$ is a hydrogen atom or a methyl group, and $R^2$ is a hydrocarbon group having 2 to 20 carbon atoms, a linear or branched alkyl group having 2 to 8 carbon atoms, in which at least one hydrogen atom is substituted with a hydroxy group, a linear or branched alkyl group having 2 to 8 carbon atoms, in which one or two hydrogen atoms are substituted with a (meth)acryloyloxy group, or a group having an etheric oxygen between carbon atoms of a hydrocarbon group having 2 to 8 carbon atoms) and a component A: $\alpha$-olefin, in which a contained amount of the component A is 1 ppm by mass or more and 1500 ppm by mass or less, and a moisture content is 5000 ppm by mass or less.

EP 4 592 323 A1

(52) Cooperative Patent Classification (CPC): (Cont.)

C-Sets
**C07C 67/03, C07C 69/54;**
**C07C 67/62, C07C 69/54**

## Description

### TECHNICAL FIELD

The present invention relates to an ester-compound-containing composition and a method for producing the same, a polymerizable composition, and a method for producing a (meth)acrylic polymer.

Priority is claimed on Japanese Patent Application No. 2022-149219, Japanese Patent Application No. 2022-149220, and Japanese Patent Application No. 2022-149221, filed September 20, 2022, the contents of which are incorporated herein by reference.

### BACKGROUND ART

A (meth)acrylic polymer obtained from a (meth)acrylic acid ester is used in various fields such as a coating material, an adhesive, a resin-reforming agent, artificial marble, and paper latex.

As a method for producing the (meth)acrylic acid ester, for example, a method in which a target (meth)acrylic acid alkyl ester is produced by an ester exchange reaction between a (meth)acrylic acid alkyl ester and an alkyl alcohol as raw materials in the presence of $Ti(OR)_4$ (R is an alkyl group) as a catalyst is known (for example, Patent Document 1).

### Citation List

### Patent Document

Patent Document 1:Japanese Unexamined Patent Application, First Publication No. 2009-274986

### SUMMARY OF INVENTION

### Technical Problem

When an ester-compound-containing composition containing a (meth)acrylic acid ester is stored for a certain period after production, quality of the ester-compound-containing composition may deteriorate.

An object of the present invention is to provide an ester-compound-containing composition having excellent storage stability and a method for producing the same, a polymerizable composition using the ester-compound-containing composition, a method for producing a (meth)acrylic polymer.

### Solution to Problem

As a result of intensive studies, the present inventors have found that a decrease in purity concentration due to generation of impurities such as a dimer of (meth)acrylic acid ester occurs during storage of the ester-compound-containing composition. The impurities such as a dimer of (meth)acrylic acid ester may cause a decrease in physical properties of a (meth)acrylic polymer.

The present inventors have further conducted studies, and have found that, when the ester-compound-containing composition contains a specific component, the generation of impurities such as a dimer of (meth)acrylic acid ester is suppressed, and storage stability is improved, thereby completing the present invention.

That is, the present invention includes the following aspects.

[1] An ester-compound-containing composition, comprising:

an ester compound (1) represented by Formula (1); and
a component A: an α-olefin,
wherein a contained amount of the component A is 1 ppm by mass or more and 1500 ppm by mass or less, and a moisture content is 5000 ppm by mass or less,

$$CH_2=CR^1\text{-}C(=O)\text{-}O\text{-}R^2 \cdots \qquad (1),$$

wherein, in Formula (1), $R^1$ is a hydrogen atom or a methyl group, and $R^2$ is a hydrocarbon group having 2 to 20 carbon atoms, which may have a heteroatom and may have a functional group.

[2] The ester-compound-containing composition according to Claim 1,

wherein $R^2$ in Formula (1) is a hydrocarbon group having 2 to 20 carbon atoms, a linear or branched alkyl group having 2 to 8 carbon atoms, in which at least one hydrogen atom is substituted with a hydroxy group, a group in which one or two hydrogen atoms in a linear or branched alkyl group or hydroxyalkyl group having 2 to 8 carbon atoms are substituted with a (meth)acryloyloxy group, or a group having an etheric oxygen between carbon atoms of a hydrocarbon group having 2 to 8 carbon atoms.

[3] The ester-compound-containing composition according to [1],

wherein the ester compound (1) includes an ester compound (1-1) represented by Formula (1-1),

$$CH_2=CR^{a1}\text{-}C(=O)\text{-}O\text{-}R^{a2} \cdots \qquad (1\text{-}1),$$

wherein, in Formula (1-1), $R^{a1}$ is a hydrogen atom or a methyl group, and $R^{a2}$ is a hydrocarbon group having 2 to 20 carbon atoms.

[4] The ester-compound-containing composition according to [3],
wherein the ester compound (1-1) in which $R^{a2}$ is a linear or branched alkyl group having 2 to 20 carbon atoms is included.

[5] The ester-compound-containing composition according to [3] or [4],
wherein a contained amount of the ester compound (1-1) is 85.00% by mass or more and 99.99% by mass or less.

[6] The ester-compound-containing composition according to any one of [1] to [5],

wherein the ester compound (1) includes one or more ester compounds (1-2) selected from the group consisting of an ester compound (1-21) represented by Formula (1-21), an ester compound (1-22) represented by Formula (1-22), and an ester compound (1-23) represented by Formula (1-23),

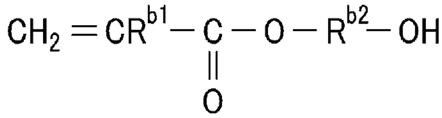

・・・(1－2 1)

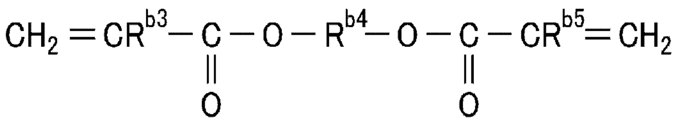

・・・(1－2 2)

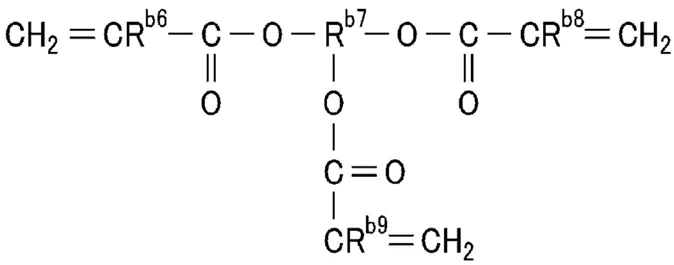

・・・(1－2 3)

wherein, in Formulae (1-21) to (1-23), $R^{b1}$, $R^{b3}$, $R^{b5}$, $R^{b6}$, $R^{b8}$, and $R^{b9}$ are each independently a hydrogen atom or a methyl group, $R^{b2}$ and $R^{b4}$ are each independently a linear or branched alkylene group or hydroxyalkylene group having 2 to 8 carbon atoms, and $R^{b7}$ is a linear or branched trivalent hydrocarbon group having 2 to 8 carbon atoms.

[7] The ester-compound-containing composition according to [6],
wherein the ester compound (1-2) includes one or more selected from the group consisting of ethylene glycol di(meth)acrylate, 2-hydroxyethyl (meth)acrylate, 3-hydroxypropyl (meth)acrylate, and trimethylolpropane tri(meth)acrylate.

[8] The ester-compound-containing composition according to [6] or [7],
wherein a contained amount of the ester compound (1-2) is 85.00% by mass or more and 99.99% by mass or less.

[9] The ester-compound-containing composition according to any one of [1] to [8],

wherein the ester compound (1) includes an ester compound (1-3) represented by Formula (1-3),

$$CH_2=CR^{c1}-C(=O)-O-R^{c2} \cdots \quad (1\text{-}3),$$

wherein, in Formula (1-3), $R^{c1}$ is a hydrogen atom or a methyl group, and $R^{c2}$ is a group having an etheric oxygen between carbon atoms of a hydrocarbon group having 2 to 8 carbon atoms.

[10] The ester-compound-containing composition according to [9],
wherein the ester compound (1-3) in which $R^{c2}$ is a 2-methoxyethyl group, a glycidyl group, or a tetrahydrofurfuryl group is included.
[11] The ester-compound-containing composition according to [9] or [10],
wherein a contained amount of the ester compound (1-3) is 85.00% by mass or more and 99.99% by mass or less.
[12] The ester-compound-containing composition according to any one of [1] to [11],
wherein the component A includes an $\alpha$-olefin having 6 or more and 12 or less carbon atoms.
[13] The ester-compound-containing composition according to any one of [1] to [12], further comprising:
a component B: a polymerization inhibitor.
[14] The ester-compound-containing composition according to any one of [1] to [13],
wherein the ester-compound-containing composition is stored for 1 day or longer.
[15] A method for producing an ester-compound-containing composition containing an ester compound (1) represented by Formula (1), the production method comprising:

performing an ester exchange reaction between methyl (meth)acrylate and an alcohol (a) having 2 to 20 carbon atoms in a presence of a component A: an $\alpha$-olefin,

$$CH_2=CR^1-C(=O)-O-R^2 \cdots \quad (1),$$

wherein, in Formula (1), $R^1$ is a hydrogen atom or a methyl group, and $R^2$ is a hydrocarbon group having 2 to 20 carbon atoms, which may have a heteroatom and may have a functional group.

[16] The method for producing an ester-compound-containing composition according to [15],

wherein $R^2$ in Formula (1) is a hydrocarbon group having 2 to 20 carbon atoms, a linear or branched alkyl group having 2 to 8 carbon atoms, in which at least one hydrogen atom is substituted with a hydroxy group, a group in which one or two hydrogen atoms in a linear or branched alkyl group or hydroxyalkyl group having 2 to 8 carbon atoms are substituted with a (meth)acryloyloxy group, or a group having an etheric oxygen between carbon atoms of a hydrocarbon group having 2 to 8 carbon atoms, and
the alcohol (a) is at least one selected from the following alcohols (a1) to (a3),
(a1) a monoalcohol having 2 to 20 carbon atoms,
(a2) one or more alcohols selected from the group consisting of a dialcohol having 2 to 8 carbon atoms and a trialcohol having 2 to 8 carbon atoms,
(a3) an ether-bond-containing alcohol having 2 to 8 carbon atoms.

[17] The method for producing an ester-compound-containing composition according to [16],

wherein an ester compound (1-1) represented by Formula (1-1) is obtained by the ester exchange reaction between the methyl (meth)acrylate and the alcohol (a1) in the presence of the component A,

$$CH_2=CR^{a1}-C(=O)-O-R^{a2} \cdots \quad (1\text{-}1),$$

wherein, in Formula (1-1), $R^{a1}$ is a hydrogen atom or a methyl group, and $R^{a2}$ is a hydrocarbon group having 2 to 20 carbon atoms.

[18] The method for producing an ester-compound-containing composition according to [17],
wherein the alcohol (a1) includes a linear or branched monoalcohol having 2 to 20 carbon atoms.
[19] The method for producing an ester-compound-containing composition according to any one of [15] to [18],

wherein an ester compound (1-2) including one or more ester compounds (1-2) selected from the group consisting of an ester compound (1-21) represented by Formula (1-21), an ester compound (1-22) represented by Formula (1-22), and an ester compound (1-23) represented by Formula (1-23) is obtained by the ester

exchange reaction between the methyl (meth)acrylate and the alcohol (a2) in the presence of the component A,

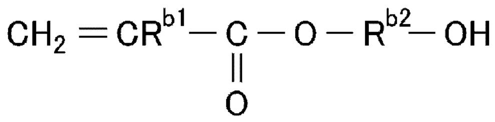

・・・(1－21)

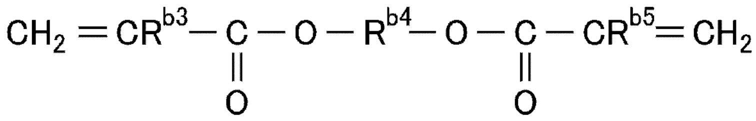

・・・(1－22)

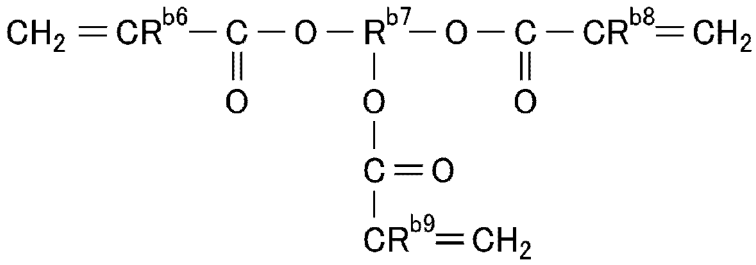

・・・(1－23)

wherein, in Formulae (2-1) to (2-3), $R^{b1}$, $R^{b3}$, $R^{b5}$, $R^{b6}$, $R^{b8}$, and $R^{b9}$ are each independently a hydrogen atom or a methyl group, $R^{b2}$ and $R^{b4}$ are each independently a linear or branched alkylene group or hydroxyalkylene group having 2 to 8 carbon atoms, and $R^{b7}$ is a linear or branched trivalent hydrocarbon group having 2 to 8 carbon atoms.

[20] The method for producing an ester-compound-containing composition according to [19],
wherein the alcohol (a2) includes one or more selected from the group consisting of ethylene glycol, 1,2-propanediol, 1,3-propanediol, and trimethylolpropane.
[21] The method for producing an ester-compound-containing composition according to any one of [15] to [20],

wherein an ester compound (1-3) represented by Formula (1-3) is obtained by the ester exchange reaction between the methyl (meth)acrylate and the alcohol (a3) in the presence of the component A,

$$CH_2{=}CR^{c1}\text{-}C({=}O)\text{-}O\text{-}R^{c2} \cdots \qquad (1\text{-}3),$$

wherein, in Formula (1-3), $R^{c1}$ is a hydrogen atom or a methyl group, and $R^{c2}$ is a group having an etheric oxygen between carbon atoms of a hydrocarbon group having 2 to 8 carbon atoms.

[22] The method for producing an ester-compound-containing composition according to [21],
wherein the alcohol (a3) includes one or more selected from the group consisting of 2-methoxyethanol, glycidyl alcohol, and tetrahydrofurfuryl alcohol.
[23] The method for producing an ester-compound-containing composition according to any one of [15] to [22],
wherein the component A includes an $\alpha$-olefin having 6 or more and 12 or less carbon atoms.
[24] A polymerizable composition for producing a (meth)acrylic polymer, comprising:
the ester-compound-containing composition according to any one of [1] to [14].
[25] The polymerizable composition according to [24],
wherein a contained amount of a component C: at least one compound selected from the group consisting of a compound of a transition metal and a compound of Group 13 element is $7 \times 10^4$ ppm by mass or less with respect to a total mass of the component A in the ester-compound-containing composition.
[26] A method for producing a (meth)acrylic polymer, comprising:
polymerizing the polymerizable composition according to [24].
[27] A method for producing a (meth)acrylic polymer, comprising:
polymerizing the polymerizable composition according to [25].

Advantageous Effects of Invention

According to the present invention, it is possible to provide an ester-compound-containing composition having excellent storage stability and a method for producing the same, a polymerizable composition using the ester-compound-containing composition, a method for producing a (meth)acrylic polymer.

DESCRIPTION OF EMBODIMENTS

Definitions of the following terms in the present specification and claims are as follows.
"Monomer" means a compound having a polymerizable carbon-carbon double bond.
"(Meth)acrylate" is selected from "acrylate" and "methacrylate".
"(Meth)acrylic acid" is selected from "acrylic acid" and "methacrylic acid".
"$\alpha$-olefin" is an olefin hydrocarbon having a carbon-carbon double bond at an $\alpha$-position.
"Transition metal" means a metal element located in Group 3 to Group 12 in the periodic table. Typically, scandium (Sc), titanium (Ti), vanadium (V), chromium (Cr), manganese (Mn), iron (Fe), cobalt (Co), nickel (Ni), copper (Cu), zinc (Zn), yttrium (Y), zirconium (Zr), niobium (Nb), molybdenum (Mo), technetium (Tc), ruthenium (Ru), rhodium (Rh), palladium (Pd), silver (Ag), cadmium (Cd), lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), hafnium (Hf), tantalum (Ta), tungsten (W), rhenium (Re), osmium (Os), iridium (Ir), platinum (Pt), gold (Au), mercury (Hg), and the like can be mentioned.
"Group 13 element" mean an element located in Group 13 in the periodic table. Typically, boron (B), aluminum (Al), gallium (Ga), indium (In), and thallium (Tl) can be mentioned.
The "periodic table" means "Periodic Table of Elements" (URL https://pubchem.ncbi.nlm.nih.gov/periodic-table/ ).

[Ester-compound-containing composition]

One example of an ester-compound-containing composition according to the embodiment of the present invention contains an ester compound (1) described later and a component A: an $\alpha$-olefin.

(Ester compound (1))

The ester compound (1) is a (meth)acrylic acid ester represented by Formula (1).

$$CH_2=CR^1-C(=O)-O-R^2 \cdots \qquad (1)$$

Here, in Formula (1), $R^1$ is a hydrogen atom or a methyl group, and $R^2$ is a hydrocarbon group having 2 to 20 carbon atoms, which may have a heteroatom and may have a functional group.
Examples of the heteroatom which may be included in $R^2$ include nitrogen, oxygen, phosphorus, and sulfur; and nitrogen or oxygen is preferable.
Examples of the functional group which may be included in $R^2$ include a hydroxy group, an aldehyde group, a carbonyl group, a carboxy group, an amino group, a nitro group, a nitroso group, a thiol group, a sulfonic acid group, a fluoro group, a chloro group, a bromo group, and an iodo group; and a hydroxy group, a carbonyl group, a carboxy group, an amino group, a thiol group, a sulfonic acid group, a fluoro group, a chloro group, or a bromo group is preferable.
$R^2$ is preferably a hydrocarbon group having 2 to 20 carbon atoms, a linear or branched alkyl group having 2 to 8 carbon atoms, in which at least one hydrogen atom is substituted with a hydroxy group, a group in which one or two hydrogen atoms in a linear or branched alkyl group or hydroxyalkyl group having 2 to 8 carbon atoms are substituted with a (meth)acryloyloxy group, or a group having an etheric oxygen between carbon atoms of a hydrocarbon group having 2 to 8 carbon atoms.
The linear or branched alkyl group having 2 to 8 carbon atoms, in which at least one hydrogen atom is substituted with a hydroxy group, is preferably a linear or branched alkyl group having 2 to 8 carbon atoms, in which one or two hydrogen atoms are substituted with a hydroxy group.
One example of the ester-compound-containing composition according to the embodiment can contain, as the ester compound (1), an ester compound (1-1) represented by Formula (1-1). The ester compound (1-1) is a (meth)acrylic acid ester in which $R^2$ in Formula (1) is a hydrocarbon group having 2 to 20 carbon atoms.

$$CH_2=CR^{a1}-C(=O)-O-R^{a2} \cdots \qquad (1\text{-}1)$$

Here, in Formula (1-1), $R^{a1}$ is a hydrogen atom or a methyl group. $R^{a2}$ is a hydrocarbon group having 2 to 20 carbon atoms.

The hydrocarbon group of $R^{a2}$ may be a saturated hydrocarbon group or an unsaturated hydrocarbon group.

The hydrocarbon group of $R^{a2}$ may be linear or branched, or may have a ring. When the hydrocarbon group of $R^{a2}$ has a ring, the ring may be an aliphatic ring or an aromatic ring.

The number of carbon atoms in the hydrocarbon group of $R^{a2}$ is 2 to 20, preferably 2 to 18 and more preferably 2 to 12.

Examples of the hydrocarbon group of $R^{a2}$ include an alkyl group having 2 to 20 carbon atoms, a cycloalkyl group having 3 to 20 carbon atoms, an alkenyl group having 2 to 20 carbon atoms, a cycloalkenyl group having 3 to 20 carbon atoms, an alkynyl group having 2 to 20 carbon atoms, an aryl group having 6 to 20 carbon atoms, and an aromatic alkyl group having 7 to 20 carbon atoms. Here, the "aromatic alkyl group" means a group in which one or more hydrogen atoms of an alkyl group are substituted with an aryl group.

Examples of the alkyl group of $R^{a2}$ include an ethyl group, a propyl group, an isopropyl group, an n-butyl group, an isobutyl group, a t-butyl group, a 2-ethylhexyl group, a lauryl group, and a stearyl group.

Examples of the cycloalkyl group of $R^{a2}$ include a cyclopropyl group, a cyclohexyl group, and an isobornyl group.

Examples of the alkenyl group of $R^{a2}$ include a vinyl group and an allyl group.

Examples of the cycloalkenyl group of $R^{a2}$ include a cyclopentenyl group, a cyclopentadienyl group, and a cyclohexenyl group.

Examples of the alkynyl group of $R^{a2}$ include a propynyl group.

Examples of the aryl group of $R^{a2}$ include a phenyl group and a naphthyl group.

Examples of the aromatic alkyl group of $R^{a2}$ include a benzyl group.

From the viewpoint that the ester compound (1-1) is relatively easy to obtain and is relatively easy to handle in terms of physical properties, $R^{a2}$ is preferably an alkyl group having 2 to 20 carbon atoms, a cycloalkyl group having 3 to 20 carbon atoms, an alkenyl group having 2 to 20 carbon atoms, an aryl group having 6 to 20 carbon atoms, or an aromatic alkyl group having 7 to 20 carbon atoms; and more preferably an ethyl group, an isopropyl group, an n-butyl group, an isobutyl group, a t-butyl group, a 2-ethylhexyl group, a lauryl group, a cyclohexyl group, an allyl group, a phenyl group, or a benzyl group.

Examples of the ester compound (1-1) include ethyl (meth)acrylate, butyl (meth)acrylate, isobutyl (meth) acrylate, t-butyl (meth)acrylate, 2-ethylhexyl (meth)acrylate, lauryl (meth)acrylate, stearyl (meth)acrylate, isobornyl (meth)acrylate, allyl (meth)acrylate, phenyl (meth)acrylate, and benzyl (meth)acrylate.

As the ester compound (1-1), from the viewpoint that the ester compound (1-1) is relatively easy to obtain and is relatively easy to handle in terms of physical properties, an alkyl (meth)acrylate in which $R^{a2}$ is a linear or branched alkyl group having 2 to 20 carbon atoms, a cycloalkyl (meth)acrylate in which $R^{a2}$ is a cycloalkyl group having 3 to 20 carbon atoms, an alkenyl (meth)acrylate in which $R^{a2}$ is an alkenyl group having 2 to 20 carbon atoms, an aryl (meth)acrylate in which $R^{a2}$ is an aryl group having 6 to 20 carbon atoms, or an aromatic alkyl (meth)acrylate in which $R^{a2}$ is an aromatic alkyl group having 7 to 20 carbon atoms is preferable; and ethyl (meth)acrylate, isopropyl (meth)acrylate, butyl (meth)acrylate, isobutyl (meth)acrylate, t-butyl (meth)acrylate, 2-ethylhexyl (meth)acrylate, lauryl (meth)acrylate, cyclohexyl (meth) acrylate, allyl (meth)acrylate, phenyl (meth)acrylate, or benzyl (meth)acrylate is more preferable.

The ester compound (1-1) may be used alone or in combination of two or more kinds thereof.

One example of the ester-compound-containing composition according to the embodiment can contain, as the ester compound (1), one or more ester compounds (1-2) selected from the group consisting of an ester compound (1-21) represented by Formula (1-21), an ester compound (1-22) represented by Formula (1-22), and an ester compound (1-23) represented by Formula (1-23). The ester compound (1-1) is a (meth)acrylic acid ester in which $R^2$ in Formula (1) is linear or branched alkyl group having 2 to 8 carbon atoms, in which at least one hydrogen atom is substituted with a hydroxy group, or a group in which one or two hydrogen atoms in a linear or branched alkyl group or hydroxyalkyl group having 2 to 8 carbon atoms are substituted with a (meth)acryloyloxy group.

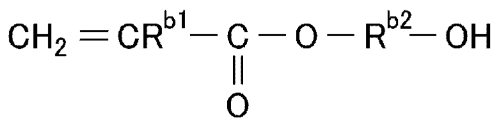

・・・(1－21)

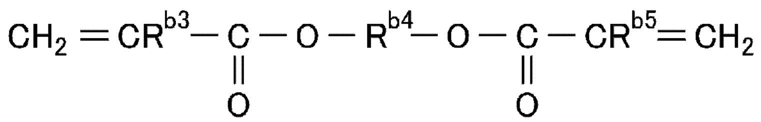

・・・(1－22)

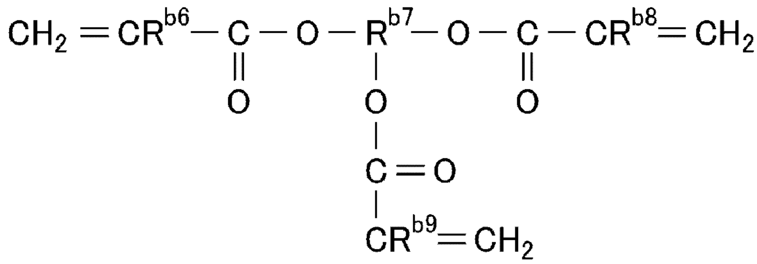

・・・(1－2 3)

Here, in Formulae (1-21) to (1-23), $R^{b1}$, $R^{b3}$, $R^{b5}$, $R^{b6}$, $R^{b8}$, and $R^{b9}$ are each independently a hydrogen atom or a methyl group, $R^{b2}$ and $R^{b4}$ are each independently a linear or branched alkylene group or hydroxyalkylene group having 2 to 8 carbon atoms, and $R^{b7}$ is a linear or branched trivalent hydrocarbon group having 2 to 8 carbon atoms.

The number of carbon atoms in the alkylene group or the hydroxyalkylene group of $R^{b2}$ and $R^{b4}$ is 2 to 8, preferably 2 to 6.

Examples of the alkylene group of $R^{b2}$ and $R^{b4}$ include an ethylene group, a propylene group, an isopropylene group, and a butylene group.

Examples of the hydroxyalkylene group of $R^{b2}$ and $R^{b4}$ include a hydroxyethylene group, a hydroxypropylene group, and a hydroxybutylene group.

The number of carbon atoms in the trivalent hydrocarbon group of $R^{b7}$ is 2 to 8, preferably 2 to 4.

Examples of the trivalent hydrocarbon group of $R^{b7}$ include $-CH_2-C(-CH_2-)(-CH_3)-CH_2-$.

Examples of the ester compound (1-21) include 2-hydroxyethyl (meth)acrylate, 2-hydroxypropyl (meth)acrylate, and 3-hydroxypropyl (meth)acrylate.

Examples of the ester compound (1-22) include ethylene glycol di(meth)acrylate, 1,3-propanediol di(meth) acrylate, 1,2-propanediol di(meth)acrylate, 1,3-butanediol di(meth)acrylate, and 1,6-hexanediol di(meth)acrylate.

Examples of the ester compound (1-23) include trimethylolpropane tri(meth)acrylate.

From the viewpoint of relatively easy availability, the ester compound (1-2) preferably includes one or more selected from the group consisting of ethylene glycol di(meth)acrylate, 2-hydroxyethyl (meth)acrylate, 3-hydroxypropyl (meth)acrylate, and trimethylolpropane tri(meth)acrylate.

The ester compound (1-2) may be used alone or in combination of two or more kinds thereof.

One example of the ester-compound-containing composition according to the embodiment can contain, as the ester compound (1), an ester compound (1-3) represented by Formula (1-3). The ester compound (1-3) is a (meth)acrylic acid ester in which $R^2$ in Formula (1) is a group having an etheric oxygen between carbon atoms of a hydrocarbon group having 2 to 8 carbon atoms.

$$CH_2=CR^{c1}-C(=O)-O-R^{c2} \cdots \qquad (1\text{-}3)$$

Here, in Formula (1-3), $R^{c1}$ is a hydrogen atom or a methyl group, and $R^{c2}$ is a group having an etheric oxygen between carbon atoms of a hydrocarbon group having 2 to 8 carbon atoms.

The number of etheric oxygens included in the hydrocarbon group of $R^{c2}$ is preferably 1, but it is not limited thereto and may be 2 or more.

The hydrocarbon group of $R^{c2}$ may be linear or branched, or may have a ring. When $R^{c2}$ has a ring, the ring may or may not include etheric oxygen.

The number of carbon atoms in the hydrocarbon group of $R^{c2}$ is 2 to 8, preferably 2 to 7.

Examples of the hydrocarbon group of $R^{c2}$ include a 2-methoxyethyl group, a 2-(2-methoxyethoxy)ethyl group, a 2-[2-(2-methoxyethoxy)ethoxy]ethyl group, a glycidyl group, and a tetrahydrofurfuryl group.

As $R^{c2}$, from the viewpoint of relatively easy availability and relatively easy handling in terms of physical properties, a 2-methoxyethyl group, a glycidyl group, or a tetrahydrofurfuryl group is preferable.

Examples of the ester compound (1-3) include 2-methoxyethyl (meth)acrylate, glycidyl (meth)acrylate, 2-(2-methoxyethoxy)ethyl (meth)acrylate, 2-[2-(2-methoxyethoxy)ethoxy]ethyl (meth)acrylate, and tetrahydrofurfuryl (meth) acrylate.

As the ester compound (1-3), from the viewpoint of relatively easy handling in terms of physical properties, 2-methoxyethyl (meth)acrylate, glycidyl (meth)acrylate, or tetrahydrofurfuryl (meth)acrylate is preferable.

The ester compound (1-3) may be used alone or in combination of two or more kinds thereof.

As the ester compound (1), only one selected from the ester compound (1-1), the ester compound (1-2), and the ester compound (1-3) may be used, or two or more thereof may be used in combination.

(Component A)

When containing the component A, a dimerization reaction of the ester compound (1) and generation of an oxidative product of the ester compound (1) are suppressed during storage, and thus an ester-compound-containing composition having excellent storage stability is obtained. The reason for this is not clear, but is presumed as follows.

During the storage of the ester-compound-containing composition, for example, a radical derived from ultraviolet rays, such as a hydroxyl radical generated by absorption of ultraviolet rays derived from sunlight by oxygen molecules, is generated. Such a radical can cause the generation of a dimer of the ester compound (1) or the generation of the oxidative product of the ester compound (1). In the α-olefin, a coupling product between olefins to which radicals generated by ultraviolet rays are added is stable, and it is presumed that a radical capture effect is excellent. Therefore, it is presumed that the α-olefin traps the radical, thereby suppressing the progress of dimerization of the ester compound (1) by a radical polymerization mechanism and the generation of the oxidative product.

The α-olefin of the component A is preferably a chain α-olefin. In addition, the component A preferably includes an α-olefin having 6 or more and 12 or less carbon atoms, and more preferably includes an α-olefin having 8 or more and 10 or less carbon atoms.

Examples of the α-olefin include 2-ethyl-1-hexene, 2-methyl-1-heptene, 4-methyl-1-heptene, 1-hexene, 1-heptene, 1-octene, 1-nonene, 1-decene, 1-undecene, and 1-dodecene.

From the viewpoint of relatively low cost, the component A is preferably at least one selected from the group consisting of 2-ethyl-1-hexene, 1-octene, and 1-dodecene, more preferably 1-octene or 1-dodecene, and still more preferably 1-octene.

The α-olefin may be used alone or in combination of two or more kinds thereof.

A proportion of the α-olefin having 6 or more and 12 or less carbon atoms with respect to the total mass of the component A is not particularly limited, but is preferably 80% by mass or more, more preferably 90% by mass or more, particularly preferably 99% by mass or more, and most preferably 100% by mass. As the proportion of the α-olefin having 6 or more and 12 or less carbon atoms becomes higher, the effect of improving the storage stability of the ester-compound-containing composition due to the α-olefin is easily obtained.

(Component B)

It is preferable that the ester-compound-containing composition according to the embodiment further contain a component B: a polymerization inhibitor, in addition to the ester compound (1) and the α-olefin.

By containing the polymerization inhibitor, the progress of the polymerization reaction of the ester compound (1) by a radical polymerization mechanism during storage is suppressed. In addition, during storage, oxygen molecules in the ester-compound-containing composition absorb ultraviolet rays derived from sunlight, thereby generating a hydroxyl radical. However, since the polymerization inhibitor can trap the hydroxyl radical, the oxidation of the ester compound (1) by the hydroxyl radical is suppressed, and the oxidative product is less likely to be generated.

Examples of the polymerization inhibitor include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound. The polymerization inhibitor used may be one or more kinds.

Examples of the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and α-nitroso-β-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include α-tocopherol and 2,3-dihydro-2,2-dimethyl-7-hydroxybenzofuran.

Examples of the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the nitrobenzene-based compound include nitrobenzene, o-dinitrobenzene, m-dinitrobenzene, p-dinitrobenzene, 2,4-dinitrobenzene, dinitrodurene, and 2,2-diphenyl-1-picrylhydrazine.

Examples of the N-oxyl compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethyl-piperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-t-butyl nitroxide, and di-t-amyl nitroxide.

Examples of the amine-based compound include N,N-diphenylamine, alkylated diphenylamine, 4,4'-dicamyldiphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, triphenylphosphite, tris(nonylphenyl) phosphite, 4,4'-isopropylidenediphenol alkylphosphite, tris(2,4-di-t-butylphenyl)phosphite, tris(biphenyl)phosphite, distearyl pentaerythritol diphosphite, di(2,4-di-t-butylphenyl)pentaerythritol diphosphite, di(nonylphenyl)pentaerythritol diphosphite, phenyl bisphenol A pentaerythritol diphosphite, tetra(tridecyl)-4,4'-butylidenebis(3-methyl-6-t-butylphenol) diphosphite, hexa(tridecyl)-1,1,3-tris(2-methyl-4-hydroxy-5-t-butylphenyl)butanetriphosphite, 3,5-dit-butyl-4-hydroxybenzyl phosphate diethyl ester, sodium-bis(4-t-butylphenyl)phosphate, sodium-2,2'-methylene-bis(4,6-di-t-butylphenyl)phosphate, and 1,3-bis(diphenoxyphosphoryloxy)benzene.

Examples of the sulfur-containing compound include diphenyl sulfide, phenothiazine, 3-oxophenothiazine, 5-oxophenothiazine, a phenothiazine dimer, 1,4-dimercaptobenzene, 1,2-dimertocaptobenzene, 2-mercaptophenol, 4-mercaptophenol, 2-(methylthio)phenol, 3,7-bis(dimethylamino)phenothiazinium chloride, and sulfur (simple substance).

Examples of the iron-containing compound include iron (III) chloride.

Examples of the copper-containing compound include copper dimethyldithiocarbamate, copper diethylthiocarbamate, copper dibutylthiocarbamate, copper salicylate, copper acetate, copper thiocyanate, copper nitrate, copper chloride, copper carbonate, copper hydroxide, copper acrylate, and copper methacrylate.

Examples of the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

As the polymerization inhibitor, from the viewpoint of easily exhibiting the effect of improving the storage stability, 4-methoxyphenol, 2,4-dimethyl-6-t-butylphenol, N,N'-dialkyl-p-phenylenediamine, phenothiazine, or 4-hydroxy-2,2,6,6-tetramethylpiperidine-N-oxyl is preferable.

The ester-compound-containing composition according to the embodiment may contain other components in addition to the ester compound (1), the component A, and the component B.

Examples of the other components include additives such as a release agent, a lubricant, a plasticizer, an antioxidant, an antistatic agent, a light stabilizer, an ultraviolet absorber, a flame retardant, a flame retardant aid, a polymerization inhibitor, a filler, a pigment, a dye, a silane coupling agent, a leveling agent, an antifoaming agent, and a fluorescent agent.

The ester-compound-containing composition according to the embodiment may contain unreacted raw materials in the production of the ester-compound-containing composition, such as methyl (meth)acrylate, alcohol, and (meth)acrylic acid.

One example of the ester-compound-containing composition according to the embodiment may contain a (meth) acrylic acid ester other than the ester compound (1).

(Formulation)

A contained amount of the ester compound (1) in the ester-compound-containing composition is preferably 85.00% by mass or more, more preferably 90.00% by mass or more, and still more preferably 95.00% by mass or more with respect to the total mass of the ester-compound-containing composition. In addition, the contained amount of the ester compound (1) is preferably 99.99% by mass or less, more preferably 99.97% by mass or less, and still more preferably 99.95% by mass or less with respect to the total mass of the ester-compound-containing composition.

The lower limit and the upper limit of the preferred contained amount of the ester compound (1) can be optionally combined, and for example, the contained amount thereof is preferably 85.00% by mass or more and 99.99% by mass or less, more preferably 90.00% by mass or more and 99.97% by mass or less, and still more preferably 95.00% by mass or more and 99.95% by mass or less.

A contained amount of the component A in the ester-compound-containing composition is 1 ppm by mass or more and 1,500 ppm by mass or less with respect to the total mass of the ester-compound-containing composition. When the contained amount of the component A is within the above-described range, an effect of suppressing a decrease in purity of the ester compound (1) due to the dimerization of the ester compound (1) and the generation of the oxidative product is obtained.

From the viewpoint that the effect of suppressing the dimerization of the ester compound (1) and the generation of the oxidative product is easily obtained, the contained amount of the component A is preferably 1 ppm by mass or more, and more preferably 3 ppm by mass or more. From the viewpoint that the amount of impurities when producing a (meth) acrylic polymer is reduced, and it is easy to suppress a decrease in physical properties of the (meth)acrylic polymer, the contained amount of the component A is preferably 1,500 ppm by mass or less, and more preferably 1,200 ppm by mass or less.

The lower limit and the upper limit of the preferred contained amount of the component A can be optionally combined, and for example, the contained amount thereof is preferably 1 ppm by mass or more and 1,500 ppm by mass or less, and more preferably 3 ppm by mass or more and 1,200 ppm by mass or less.

A contained amount of the component B in the ester-compound-containing composition is preferably 1 ppm by mass or more, more preferably 3 ppm by mass or more, and still more preferably 5 ppm by mass or more with respect to the total mass of the ester-compound-containing composition. In a case where the contained amount of the component B is the above-described lower limit value or more, the effect of suppressing the dimerization of the ester compound (1) and the generation of the oxidative product is easily obtained. The contained amount of the component B is preferably 1,500 ppm by mass or less, more preferably 1,200 ppm by mass or less, and still more preferably 1,000 ppm by mass or less with respect to the total mass of the ester-compound-containing composition. In a case where the contained amount of the component B is the above-described upper limit value or less, the amount of impurities when producing a (meth)acrylic polymer is reduced, and it is easy to suppress a decrease in physical properties of the (meth)acrylic polymer.

The lower limit and the upper limit of the preferred contained amount of the component B can be optionally combined, and for example, the contained amount thereof is preferably 1 ppm by mass or more and 1,500 ppm by mass or less, more preferably 3 ppm by mass or more and 1,200 ppm by mass or less, and still more preferably 5 ppm by mass or more and 1,000 ppm by mass or less.

The moisture content of the ester-compound-containing composition needs to be 5,000 ppm by mass or less. This is because the physical properties of the (meth)acrylic polymer are adversely affected.

The moisture content of the ester-compound-containing composition is preferably 5,000 ppm by mass or less, more preferably 4,000 ppm by mass or less, and still more preferably 3,000 ppm by mass or less.

(Analysis of ester-compound-containing composition)

The component A and the component B contained in the ester-compound-containing composition can be measured by, for example, GC-MS measurement.

**In** a GC-MS chart of the ester-compound-containing composition, when a peak is present at the same retention time as a sample of the component A and an m/z value detected in a mass spectrum of the peak matches exact mass of the component A, it can be determined that the ester-compound-containing composition contains the component A. When a sample of the component A cannot be obtained, when a pattern of the mass spectrum of the peak appearing in the GC-MS chart of the ester-compound-containing composition and a pattern of a mass spectrum of the component A in mass spectrum database and match each other, it can be determined that the peak is the peak of the component A and the ester-compound-containing composition contains the component A. Examples of the mass spectrum database include NIST 20,

NIST 17, NIST 14, and NIST 14s. In addition, when volatility is low and the detection cannot be carried out by the GC-MS measurement, the detection can be carried out by LC-MS. The component B can also be confirmed by the same method as that for the component A.

The contained amount of the ester compound (1) can be calculated by performing GC-FID measurement of the ester-compound-containing composition, quantifying by an area percentage method, and correcting the quantified moisture content using a Karl Fischer moisture meter.

The contained amount of the component A can be quantified, for example, by performing GC measurement of the ester-compound-containing composition and using an internal standard method. When a sample of the component A cannot be obtained and the component A cannot be quantified by the internal standard method, the contained amount of the component A can be calculated using the following expression by performing GC-FID measurement on any organic compound having a known contained amount.

$$\text{Contained amount of component A (ppm by mass)} = \frac{N}{N_A} \times \frac{S_A}{S} \times M$$

In the expression, N is the number of carbon atoms in one molecule of any organic compound, $N_A$ is the number of carbon atoms in one molecule of the component A, $S_A$ is a peak area of the component A, S is a peak area of the organic compound, and M is a contained amount (ppm by mass) of any organic component.

When the volatility is low and the quantification cannot be performed based on the GC area, the quantification can be performed using a chromatography method such as LC.

The contained amount of the component B can also be calculated by the same method as that for the component A.

The moisture content of the ester-compound-containing composition can be confirmed by the Karl Fischer method.

[Method for producing ester-compound-containing composition]

The ester-compound-containing composition according to the embodiment of the present invention can be produced, for example, by performing an ester exchange reaction between methyl (meth)acrylate and an alcohol (a) having 2 to 20 carbon atoms in the presence of the component A.

As the alcohol (a), at least one selected from the following alcohols (a1) to (a3) is preferably used.

(a1) a monoalcohol having 2 to 20 carbon atoms
(a2) one or more alcohols selected from the group consisting of a dialcohol having 2 to 8 carbon atoms and a trialcohol having 2 to 8 carbon atoms
(a3) an ether-bond-containing alcohol having 2 to 8 carbon atoms

Among (meth)acrylic acid esters, the methyl (meth)acrylate tends to particularly easily undergo dimerization or oxidation to produce methyl pyruvate. However, by performing the ester exchange reaction in the presence of the component A, the dimerization of methyl (meth)acrylate or the generation of methyl pyruvate is suppressed, and as a result, the yield of the ester compound (1) is improved.

The ester-compound-containing composition containing the ester compound (1-1) can be produced by performing an ester exchange reaction between methyl (meth)acrylate and the alcohol (a1) in the presence of the component A.

More specifically, for example, in a reaction vessel, the methyl (meth)acrylate is subjected to an ester exchange reaction with the alcohol (a1) as represented by Formula (2) in the presence of a catalyst and the component A. Here, $R^{a1}$ and $R^{a2}$ in Formula (2) are the same as $R^{a1}$ and $R^{a2}$ in Formula (1-1).

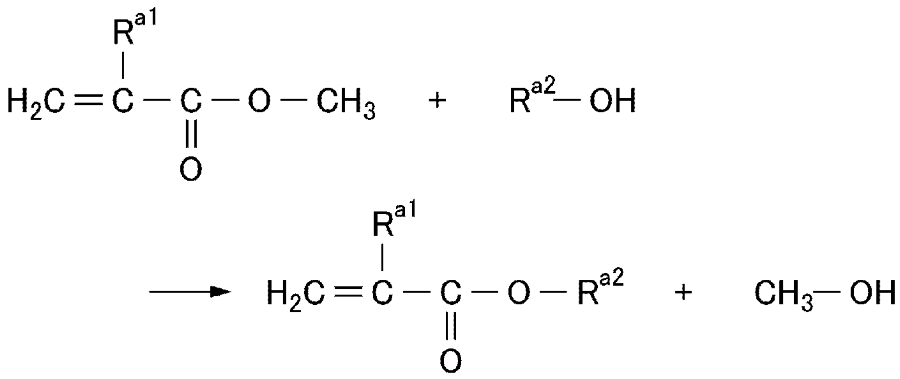

(1 − 1)

・・・(2)

Examples of the alcohol (a1) include ethanol, n-butanol, isobutanol, t-butanol, 2-ethylhexanol, lauryl alcohol, stearyl alcohol, isobornyl alcohol, allyl alcohol, phenol, and benzyl alcohol. The alcohol (a1) may be used alone or in combination of two or more kinds thereof.

The alcohol (a1) preferably includes a linear or branched monoalcohol having 2 to 20 carbon atoms.

A ratio of the amount of the methyl (meth)acrylate to be charged and the amount of the alcohol (a1) to be charged can be appropriately determined. From the viewpoint of improving productivity, the ratio of the methyl (meth)acrylate to 1 mol of the alcohol (a1) is preferably 0.1 mol or more and 10 mol or less, and more preferably 0.3 mol or more and 4 mol or less.

The ester-compound-containing composition containing the ester compound (1-2) can be produced by performing an ester exchange reaction between methyl (meth)acrylate and the alcohol (a2) in the presence of the component A.

More specifically, for example, in a reaction vessel, the methyl (meth)acrylate is subjected to an ester exchange reaction with the alcohol (a2) as represented by Formula (3) or Formula (4) in the presence of a catalyst and the component A.

Here, $R^{b1}$ to $R^{b3}$ and $R^{b5}$ to $R^{b7}$ in Formula (3) and Formula (4) are the same as $R^{b1}$ to $R^{b3}$ and $R^{b5}$ to $R^{b7}$ in Formulae (1-21) to (1-23). In addition, $R^{b41}$ is a linear or branched alkylene group having 2 to 8 carbon atoms, and $R^{b42}$ is a linear or branched hydroxyalkylene group having 2 to 8 carbon atoms.

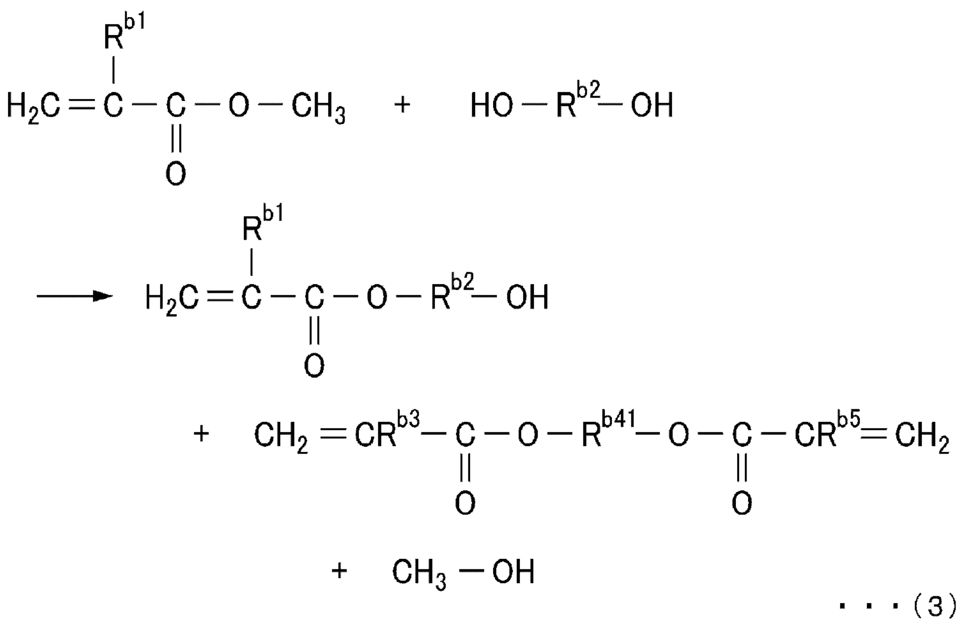

・・・(3)

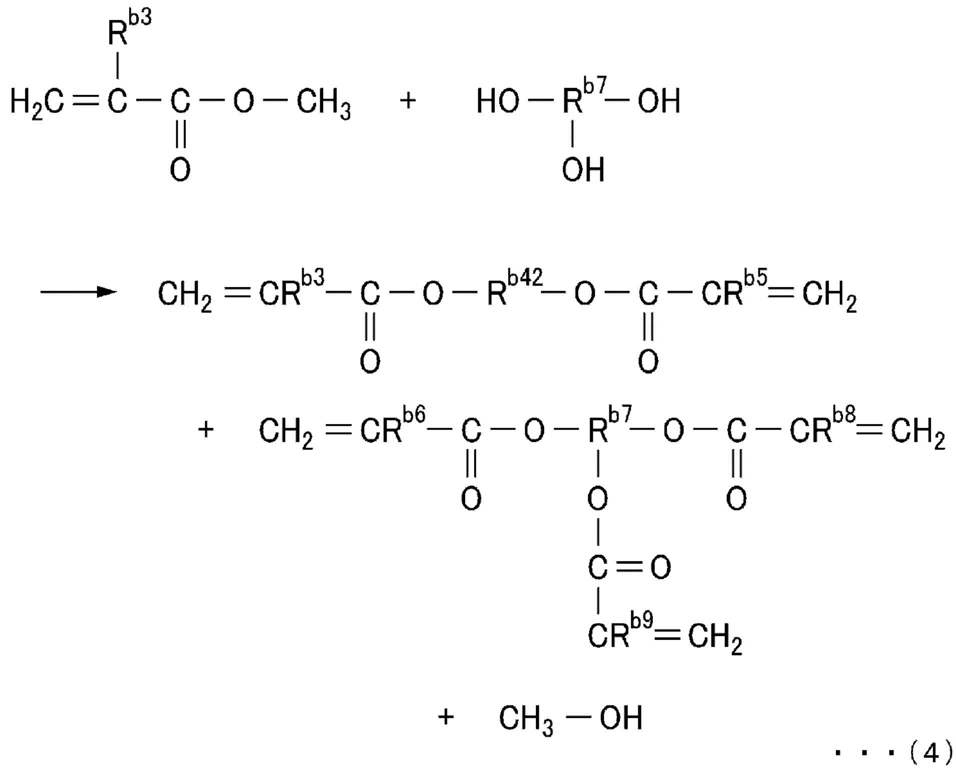

・・・(4)

Examples of the alcohol (a2) include ethylene glycol, 1,2-propanediol, 1,3-propanediol, 1,3-butanediol, 1,6-hexanediol, and trimethylolpropane. Among these, it is preferable that the alcohol (a2) include one or more selected from the group consisting of ethylene glycol, 1,2-propanediol, 1,3-propanediol, and trimethylolpropane.

The alcohol (a2) may be used alone or in combination of two or more kinds thereof.

A ratio of the amount of the methyl (meth)acrylate to be charged and the amount of the alcohol (a2) to be charged can be appropriately determined. From the viewpoint of improving productivity, the ratio of the methyl (meth)acrylate to 1 mol of the alcohol (a2) is preferably 0.1 mol or more and 10 mol or less, and more preferably 0.3 mol or more and 4 mol or less.

The ester-compound-containing composition containing the ester compound (1-3) can be produced by performing an ester exchange reaction between methyl (meth)acrylate and the alcohol (a3) in the presence of the component A.

More specifically, for example, in a reaction vessel, the methyl (meth)acrylate is subjected to an ester exchange reaction with the alcohol (a3) as represented by Formula (5) in the presence of $Ti(OR)_4$ as a catalyst (R is an alkyl group) and the component A. Here, $R^{c1}$ and $R^{c2}$ in Formula (5) are the same as $R^{c1}$ and $R^{c2}$ in Formula (1-3).

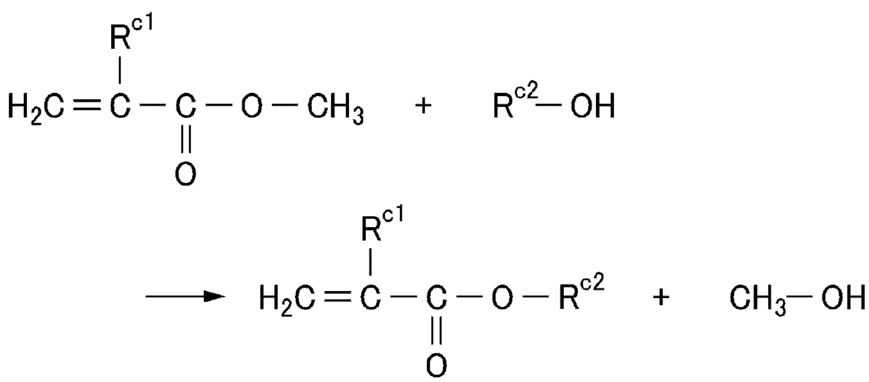

(1－3)

・・・(5)

The number of ether bonds in the alcohol (a3) is preferably 1, but is not limited thereto.

Examples of the alcohol (a3) include 2-methoxyethanol, glycidyl alcohol, and tetrahydrofurfuryl alcohol. The alcohol (a3) may be used alone or in combination of two or more kinds thereof.

The alcohol (a3) preferably includes one selected from 2-methoxyethanol, glycidyl alcohol, and tetrahydrofurfuryl alcohol.

A ratio of the amount of the methyl (meth)acrylate to be charged and the amount of the alcohol (a3) to be charged can be appropriately determined. From the viewpoint of improving productivity, the ratio of the methyl (meth)acrylate to 1 mol of the alcohol (a3) is preferably 0.1 mol or more and 10 mol or less, and more preferably 0.3 mol or more and 4 mol or less.

An ester-compound-containing composition containing two or more of the ester compounds (1-1) to (1-3) may be produced by performing an ester exchange reaction between methyl (meth)acrylate and two or more selected from the alcohols (a1) to (a3) in the presence of the component A.

In this case, a ratio of the amount of the methyl (meth)acrylate to be charged and the amount of the alcohols (a) to be charged can be appropriately determined. From the viewpoint of improving productivity, the ratio of the methyl (meth) acrylate to 1 mol of the alcohol (a) is preferably 0.1 mol or more and 10 mol or less, and more preferably 0.3 mol or more and 4 mol or less.

The reaction vessel is preferably a reaction vessel including a distillation column. Since the ester exchange reaction is an equilibrium reaction, productivity is improved by separating a by-produced methanol by the distillation column. For example, it is preferable to perform the ester exchange reaction while separating methanol as a azeotropic mixture with the methyl (meth)acrylate to the outside of the system.

Examples of the reaction vessel include a reaction vessel including a distillation column provided on an upper part of a reaction container called a reaction kettle, and a distillation column in which a distillation can can be used as a reaction container. Examples of the distillation column include a packed column-type distillation column and a tray-type distillation column.

From the viewpoint of high separation ability and stable operation, a theoretical number of column plates of the distillation column is preferably 5 or more, and more preferably 7 or more.

The catalyst to be used is not particularly limited, and examples thereof include hydroxides, carbonates, and bicarbonates of an alkali metal such as lithium, sodium, and potassium; oxides, hydroxides, and carbonates of an alkaline earth metal such as magnesium and calcium; alkali metal alkoxides such as lithium methoxide, sodium methoxide, sodium ethoxide, and potassium t-butoxide; alkali metal amides such as lithium amide, sodium amide, and potassium amide; titanium alkoxides such as tetramethyl titanate, tetraethyl titanate, tetrapropyl titanate, triisopropyl titanate, tetrabutyl titanate, and tetra(2-ethylhexyl) titanate; and tin-based compounds such as dibutyl tin oxide and dioctyl tin oxide. Among these, from the viewpoint of small amount of by-products of Michael addition reaction products during the ester exchange reaction and high catalytic activity, an alkoxide of titanium, dibutyltin oxide, or dioctyltin oxide is preferable. The catalyst may be used alone or in combination of two or more kinds thereof.

The catalyst can be supplied alone to the reaction vessel, but the catalyst can also be supplied to the reaction vessel in a state of being dissolved in the same alcohol as the alcohol (a) as the raw material, or in a state of being dissolved in (meth)acrylic acid ester as the raw material. Examples thereof include a method of directly dissolving the catalyst in the total amount of the alcohol (a) used in the reaction and supplying the catalyst to the reaction vessel, and a method of dissolving the catalyst in a part of the alcohol (a) used in the reaction and supplying the catalyst to the reaction vessel.

The amount of the catalyst used is preferably 0.001 mol% or more and 1 mol% or less, and more preferably 0.01 mol% or more and 0.1 mol% or less with respect to 1 mol of the alcohol (a).

A solvent may be used for the ester exchange reaction. When a solvent is used, it is preferable to use a solvent which forms an azeotropic formulation with the by-produced methanol.

Examples of the solvent include n-pentane, n-hexane, n-heptane, n-octane, 2,3-dimethylbutane, 2,5-dimethylhexane, 2,2,4-trimethylpentane, cyclohexane, benzene, and toluene. Among these, n-hexane, n-heptane, or cyclohexane is preferable. The solvent may be used alone or in combination of two or more thereof.

A reaction temperature for the ester exchange reaction varies depending on the type of the alcohol (a) or the solvent, but is preferably 60°C or higher and 150°C or lower.

A reaction pressure for the ester exchange reaction is not particularly limited, and the reaction may be carried out under any pressure of reduced pressure, normal pressure, or elevated pressure.

A form of the ester exchange reaction is not particularly limited, and the ester exchange reaction can be performed by a generally used method, for example, a batch method, a continuous method, or the like.

After the ester exchange reaction, unreacted raw materials and by-products may be separated by purifying the reaction solution. For the purification, for example, a known method such as distillation, crystallization, extraction, and column chromatography can be performed.

The ester exchange reaction may be performed by blending the component B into the reaction solution. When the component B is present in addition to the component A, the dimerization of methyl (meth)acrylate or the generation of methyl pyruvate is further suppressed, and thus an effect of improving the yield of the ester compound (1) is more easily obtained.

The component B may be blended into a solution containing the ester compound (1) after the ester exchange

reaction and the component A. In this case, a solution (B solution) containing the ester compound (1) and the component B is prepared separately from the solution (A solution) containing the ester compound (1) and the component A, and the A solution and the B solution are mixed with each other to obtain the ester-compound-containing composition. In addition, the ester compound (1), the A solution, and the B solution may be mixed with each other to obtain the ester-compound-containing composition.

The method for producing an ester-compound-containing composition according to the embodiment of the present invention is not limited to the above-described method.

For example, other (meth)acrylic acid esters other than the methyl (meth)acrylate may be used as the raw material instead of the methyl (meth)acrylate.

An esterification reaction between the alcohol (a) and (meth)acrylic acid may be carried out to produce the ester compound (1).

(Polymerizable composition)

The polymerizable composition according to the embodiment of the present invention is a polymerizable composition for producing a (meth)acrylic polymer, and contains the ester-compound-containing composition according to the embodiment of the present invention.

As an example of the embodiment, an ester-compound-containing composition stored for 1 day or more after production can be used as the polymerizable composition.

A storage time of the ester-compound-containing composition can be 1 day or more, 3 days or more, 7 days or more, or 14 days or more. In addition, the storage time of the ester-compound-containing composition may be 180 days or less, 150 days or less, 120 days or less, or 90 days or less. The "storage time" means an elapsed time from immediately after the production, which is not limited to a time during which the product is left to stand under a specific environment and also includes a time of transportation or the like.

A material of a storage container for the ester-compound-containing composition is not particularly limited, and for example, a metal container such as stainless steel, a resin container, or a glass container can be used. A transparent container or an opaque container can be used.

A temperature at the time of storing the ester-compound-containing composition is preferably -10°C or higher and more preferably 0°C or higher, and is preferably 60°C or lower and more preferably 50°C or lower. By setting the temperature to -10°C or higher, a load on a cooling device can be reduced, and by setting the temperature to 60°C or lower, it is easy to suppress the generation of the dimerized substance or the oxidative product of (meth)acrylic acid ester.

An oxygen concentration of a gas phase portion at the time of storing the ester-compound-containing composition is preferably 5% by volume or more and more preferably 7% by volume or more, and is preferably 30% by volume or less and more preferably 22% by volume or less. By setting the oxygen concentration of the gas phase portion to 5% by volume or more, it is easy to prevent the ester compound from being unintentionally polymerized by a polymerization prevention effect of oxygen, and by setting the oxygen concentration of the gas phase portion to 30% by volume or less, it is easy to suppress oxidation of the (meth)acrylic acid ester by oxygen and the generation of various impurities.

In the embodiment, the ester-compound-containing composition after the storage may be used in the polymerizable composition without further blending a monomer, or other monomer copolymerizable with the ester compound (1) may be further blended with the ester-compound-containing composition after the storage to obtain the polymerizable composition.

As an example of the embodiment, the ester-compound-containing composition after the production may be stored in a state in which the other monomer copolymerizable with the ester compound (1) is blended, and used in the polymerizable composition. In this case, it is preferable that a polymerization initiator not be blended during the storage.

A proportion of the ester compound (1) to the total mass of monomers in the polymerizable composition is preferably 10% by mass or more and more preferably 20% by mass or more, and preferably 90% by mass or less and more preferably 80% by mass or less.

Examples of the other monomer copolymerizable with the ester compound (1) include methyl (meth)acrylate, unsaturated carboxylic acid, unsaturated carboxylic acid anhydride, maleimide, a hydroxy group-containing vinyl monomer, vinyl ester, a nitrogen-containing vinyl monomer, an epoxy group-containing monomer, an aromatic vinyl monomer, alkanediol di(meth)acrylate, polyoxyalkylene glycol di(meth)acrylate, and a vinyl monomer having two or more ethylenically unsaturated bonds in the molecule.

Examples of the unsaturated carboxylic acid include acrylic acid, methacrylic acid, maleic acid, and itaconic acid.

Examples of the unsaturated carboxylic acid anhydride include maleic acid anhydride and itaconic acid anhydride.

Examples of the maleimide include N-phenylmaleimide and N-cyclohexylmaleimide.

Examples of the hydroxy group-containing vinyl monomer include 2-hydroxyethyl acrylate, 2-hydroxyethyl methacrylate, and 2-hydroxypropyl methacrylate.

Examples of the vinyl ester include vinyl acetate and vinyl benzoate.

Examples of the nitrogen-containing vinyl monomer include methacrylamide and acrylonitrile.

Examples of the epoxy group-containing monomer include glycidyl acrylate and glycidyl methacrylate.

Examples of the aromatic vinyl monomer include styrene and $\alpha$-methylstyrene.

Examples of the alkanediol di(meth)acrylate include ethylene glycol di(meth)acrylate, 1,2-propylene glycol di(meth)acrylate, 1,3-butylene glycol di(meth)acrylate, and 1,6-hexanediol di(meth)acrylate.

Examples of the polyoxyalkylene glycol di(meth)acrylate include diethylene glycol di(meth)acrylate, dipropylene glycol di(meth)acrylate, triethylene glycol (meth)acrylate, tetraethylene glycol di(meth)acrylate, polyethylene glycol di(meth)acrylate, and neopentyl glycol di(meth)acrylate.

Examples of the vinyl monomer having two or more ethylenically unsaturated bonds in the molecule include divinylbenzene.

As the other monomer, vinyl chloride, vinylidene chloride, derivatives thereof, an unsaturated polyester prepolymer obtained from at least one polycarboxylic acid including an ethylenically unsaturated polycarboxylic acid and at least one diol, and a vinyl ester prepolymer obtained by acrylic modification of a terminal of an epoxy group may be used.

The other monomer may be used alone or in combination of two or more kinds thereof.

The polymerizable composition according to the embodiment preferably contains a polymerization initiator.

Examples of the polymerization initiator include an azo compound, an organic peroxide, a persulfate compound, and a redox polymerization initiator. The polymerization initiator may be used alone or in combination of two or more thereof.

Examples of the azo compound include 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate.

Examples of the organic peroxide include benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxy-isononanoate, di-t-butyl peroxide, di-t-hexyl peroxide, lauroyl peroxide, and dilauroyl peroxide.

Examples of the persulfate compound include potassium persulfate.

An addition amount of the polymerization initiator is not particularly limited, and for example, can be 0.005 to 5 parts by mass with respect to 100 parts by mass of the total mass of the monomers in the polymerizable composition.

In the polymerizable composition according to the embodiment, a contained amount of a component C: at least one compound selected from the group consisting of a compound of a transition metal and a compound of Group 13 element is $7 \times 10^4$ ppm by mass or less with respect to the total mass of the component A. In this manner, weather fastness of a (meth)acrylic polymer to be obtained is improved, and yellowing is suppressed.

The reason for this is considered to be as follows.

The $\alpha$-olefin which is the component A cannot provide a resonance stabilization effect as compared with the ester compound (1) which is a conjugated monomer, and thus has a significantly low reactivity. Therefore, unless a specific polymerization catalyst such as the component C is used under special conditions which exhibit the effect of the catalyst, unreacted $\alpha$-olefin remains in the obtained (meth)crylic polymer. It is considered that the (meth)acrylic polymer in which unreacted $\alpha$-olefin remains has favorable weather fastness and suppressed yellowing.

A contained amount of the component C is preferably $7 \times 10^4$ ppm by mass or less, more preferably $1 \times 10^4$ ppm by mass or less, still more preferably 1,000 ppm by mass or less, and particularly preferably 0 ppm by mass. Here, the "0 ppm by mass" indicates that the component C is below the detection limit in the GC-MS measurement.

Examples of the component C include a compound of a transition metal of Group 5 to Group 12, having a chelating ligand, and a Lewis acid catalyst.

Specific examples of the transition metal include vanadium, niobium, tantalum, chromium, molybdenum, tungsten, manganese, iron, platinum, ruthenium, cobalt, rhodium, nickel, palladium, and copper. Among these, as the transition metal, a transition metal of Group 8 to Group 11 is preferable, a transition metal of Group 10 is more preferable, and nickel or palladium is still more preferable. One kind of these transition metals may be used alone, or two or more kinds thereof may be used in combination.

The chelating ligand has at least two atoms selected from the group consisting of P, N, O, and S, includes a ligand which is bidentate or multidentate, and is electronically neutral or anionic. A review by Ittel and the like illustrates a structure of the chelating ligand (Ittel et al., "Late-Metal Catalysts for Ethylene Homo- and Copolymerization", Chemical Reviews, March 25, 2000, Vol. 100, No. 4, pp. 1169 to 1204).

Examples of the chelating ligand include a bidentate anionic P and O ligand. Examples of the above-described

bidentate anionic P and O ligand include phosphorus sulfonic acid, phosphorus carboxylic acid, phosphorus phenol, and phosphorus enolate. Examples of a chelating ligand other than the above-described bidentate anionic P and O ligand include bidentate anionic N and O ligands. Examples of the above-described bidentate anionic N and O ligand include salicylaldominate and pyridinecarboxylic acid. Examples of a chelating ligand other than the above-described bidentate anionic P and O ligand and the above-described bidentate anionic N and O ligand include diimine ligands, diphenoxide ligands, and diamide ligands.

As a catalyst which is the compound of a transition metal of Group 5 to Group 11, having a typical chelating ligand, catalysts such as SHOP catalysts and Drent catalysts are known. The SHOP catalyst is a catalyst in which a phosphorus-based ligand having an aryl group which may have a substituent is coordinated to a nickel metal. In addition, the Drent catalyst is a catalyst in which a phosphorus-based ligand having an aryl group which may have a substituent is coordinated to a palladium metal.

In addition, examples of a typical Lewis acid catalyst include a cationic complex of dihydric palladium or platinum. The above-described cationic complex of dihydric palladium or platinum exhibits Lewis acidity, and is useful as a Lewis acid catalyst for Diels-Alder reaction and the like. In addition, a compound of boron and aluminum in Group 13 element, a compound of titanium as a transition metal in the 4th period, a compound of zirconium as a transition metal in the 5th period, and the like are also preferable because they exhibit Lewis acidity.

As an example of the embodiment, the ester-compound-containing composition after the storage may be blended with various additives such as a release agent, a lubricant, a plasticizer, an antioxidant, an antistatic agent, a light stabilizer, an ultraviolet absorber, a flame retardant, a flame retardant assistant, a polymerization inhibitor, a filler, a pigment, a dye, a silane coupling agent, a leveling agent, an antifoaming agent, a fluorescent agent, and a chain transfer agent, as necessary.

[Method for producing (meth)acrylic polymer]

A (meth)acrylic polymer can be produced by polymerizing the polymerizable composition according to the embodiment of the present invention.

The polymerization method is not particularly limited, and examples thereof include a bulk polymerization method, a solution polymerization method, an emulsion polymerization method, and a suspension polymerization method. From the viewpoint of environmental load due to the use of the solvent or the like and viewpoint of transparency of the obtained (meth)acrylic polymer, a bulk polymerization method is preferable.

Specific methods of the bulk polymerization method are not particularly limited, and examples thereof include known casting polymerization methods such as a cell casting method and a continuous casting method.

A polymerization temperature is preferably 125°C or higher and 210°C or lower, and more preferably 130°C or higher and 180°C or lower.

A polymerization time is preferably 0.5 hours or more and 24 hours or less.

A weight-average molecular weight (Mw) of the (meth)acrylic polymer is not particularly limited, and can be, for example, 100,000 or more and 1,000,000 or less. As the Mw of the (meth)acrylic polymer becomes higher, solvent resistance and chemical resistance can be further improved.

The Mw of the (meth)acrylic polymer can be controlled by adjusting the polymerization temperature, the polymerization time, the addition amount of the polymerization initiator, and the like.

(Formulation)

A contained amount of the ester compound (I) in the ester-compound-containing composition is preferably 85.00% by mass or more, more preferably 90.00% by mass or more, and still more preferably 95.00% by mass or more with respect to the total mass of the ester-compound-containing composition. In addition, the contained amount of the ester compound (I) is preferably 99.99% by mass or less, more preferably 99.97% by mass or less, and still more preferably 99.95% by mass or less with respect to the total mass of the ester-compound-containing composition.

The lower limit and the upper limit of the preferred contained amount of the ester compound (I) can be optionally combined, and for example, the contained amount thereof is preferably 85.00% by mass or more and 99.99% by mass or less, more preferably 90.00% by mass or more and 97.97% by mass or less, and still more preferably 95.00% by mass or more and 99.95% by mass or less.

A proportion of the ester compound (I) to the total mass of monomers in the polymerizable composition is preferably 10% by mass or more and more preferably 20% by mass or more, and preferably 90% by mass or less and more preferably 80% by mass or less.

Examples of the other monomer copolymerizable with the ester compound (I) include methyl (meth)acrylate, unsaturated carboxylic acid, unsaturated carboxylic acid anhydride, maleimide, vinyl ester, a nitrogen-containing vinyl monomer, an epoxy group-containing monomer, an aromatic vinyl monomer, polyoxyalkylene glycol di(meth)acrylate, and

a vinyl monomer having two or more ethylenically unsaturated bonds in the molecule.

The present invention will be specifically described below with reference to Examples, but the present invention is not limited by the following description.

<Ester-compound-containing composition containing ester compound (1-1)>

[GC-MS measurement]

The analysis of the ester-compound-containing composition after storage was carried out using GC-MS, and a formulation was determined by the method described in the section of "Analysis of ester-compound-containing composition" above. QP-2010 Ultra manufactured by Shimadzu Corporation was used for the GC-MS measurement.

(GC conditions)

Column (product name: InertCap1, manufactured by GL Sciences Inc.)
length: 60 m, inner diameter: 0.25 mm, film thickness: 1.50 μm
Injection volume: 1.0 μL
Vaporization chamber temperature: 280°C
Column oven temperature: held at 150°C for 1 minute, raised from 150°C to 260°C at 2.5 °C/min, and held at 260°C for 15 minutes
Carrier gas: helium
Injection mode: split (split ratio: 25)
Control mode: constant linear velocity (35.0 cm/sec)
Pressure: 262.8 KPa
Total flow rate: 47.2 mL/min
Purge flow rate: 3.0 mL/min
Column flow rate: 1.70 mL/min

(MS conditions)

Ionization method: electron ionization (EI)
Ion source temperature: 200°C
Interface temperature: 300°C
m/z detection range: 30 to 600
Detection time: 60 minutes

[Moisture content]

A moisture content was quantified by the Karl Fischer method using a trace moisture content measuring device (product name: CA-200, manufactured by Nittoseiko Analytech Co., Ltd.).

[Example A1]

0.0208 g of 1-octane as a component A was added to 10.0014 g of butyl methacrylate (BMA, manufactured by Tokyo Chemical Industry Co., Ltd., moisture content: 164 ppm by mass) to prepare an A solution.

In addition, 0.0200 g of 2,4-dimethyl-6-t-butylphenol as a component B was added to 10.0089 g of BMA (moisture content: 164 ppm by mass) to prepare a B solution.

Next, 0.0276 g of the A solution and 0.0297 g of the B solution were added to 4.9998 g of BMA (moisture content: 164 ppm by mass) to prepare an ester-compound-containing composition.

The obtained ester-compound-containing composition was stored at 25°C for 21 days, and a generated amount (ppm by mass) of BMA dimer was calculated from a proportion (area%) of an area value of BMA dimer to the total area value of all components detected by the GC-MS measurement. In addition, a purity (%) of BMA was calculated from the proportion (area%) of the area value of BMA to the total area value of all detected components. In the calculation, a value obtained by subtracting the moisture content (% by mass) from 100% was multiplied by the area% of each component to

calculate the BMA dimer and the purity of BMA.

[Examples A2 to A10 and Comparative Example A1]

An ester-compound-containing composition was prepared in the same manner as in Example A1, except that the formulations of the A solution, the B solution, and the ester-compound-containing composition were changed as shown in Table 1, and the generated amount of BMA dimer and the purity of BMA were obtained.

[Table 1]

| | A solution | | | | B solution | | | | Ester-compound-containing composition | | |
|---|---|---|---|---|---|---|---|---|---|---|---|
| | BMA [g] | Component A | | | BMA [g] | Component B | | | BMA [g] | A solution [g] | B solution [g] |
| | | Compound name | Addition amount [g] | Contained amount [ppm by mass] | | Compound name | Addition amount [g] | Contained amount [ppm by mass] | | | |
| Example A1 | 10.0014 | Oct | 0.0208 | 2080 | 10.0089 | DMTBP | 0.0200 | 1998 | 4.9998 | 0.0276 | 0.0297 |
| Example A2 | 10.0037 | 2EH | 0.0212 | 2119 | 10.0089 | DMTBP | 0.0200 | 1998 | 5.0024 | 0.0302 | 0.0276 |
| Example A3 | 10.0018 | Ddc | 0.0221 | 2210 | 10.0089 | DMTBP | 0.0200 | 1998 | 4.9999 | 0.0290 | 0.0368 |
| Example A4 | 10.0014 | Oct | 0.0208 | 2080 | 10.0038 | 4MP | 0.0198 | 1979 | 5.0028 | 0.0321 | 0.0253 |
| Example A5 | 10.0014 | Oct | 0.0208 | 2080 | 10.0041 | IPPPD | 0.0212 | 2119 | 5.0017 | 0.0276 | 0.0273 |
| Example A6 | 10.0014 | Oct | 0.0208 | 2080 | 10.0013 | PHT | 0.0238 | 2380 | 5.0019 | 0.0275 | 0.0250 |
| Example A7 | 10.0014 | Oct | 0.0208 | 2080 | 10.001 | HO-TEMPO | 0.0220 | 2200 | 5.0048 | 0.0293 | 0.0316 |
| Example A8 | 10.0014 | Oct | 0.0208 | 2080 | 10.0089 | DMTBP | 0.0200 | 1998 | 4.7533 | 0.2604 | 0.0248 |
| Example A9 | 10.0014 | Oct | 0.0208 | 2080 | 10.0089 | DMTBP | 0.0200 | 1998 | 4.7563 | 0.0257 | 0.0295 |
| Example A10 | 10.0014 | Oct | 0.0208 | 2080 | 10.0089 | - | - | - | 5.0079 | 0.0273 | - |
| Comparative Example A1 | - | - | - | - | - | - | - | - | 5.0089 | - | - |

Abbreviations in Table 1 have the following meanings.

BMA: butyl methacrylate

Oct: 1-octene

2EH: 2-ethyl-1-hexene

Ddc: 1-dodecene

DMTBP: 2,4-dimethyl-6-t-butylphenol

4MP: 4-methoxyphenol

IPPPD: N-isopropyl-N'-phenyl-p-phenylenediamine

PHT: phenothiazine

HO-TEMPO: 4-hydroxy-2,2,6,6-tetramethylpiperidine-N-oxyl

Table 2 shows the measurement results of the generated amount of BMA dimer and the purity of BMA in each example.

[Table 2]

| | Formulation of ester-compound-containing composition (calculated value) | | | | Analysis result of ester-compound-containing composition after storage | |
|---|---|---|---|---|---|---|
| | BMA [% by mass] | Component A [ppm by mass] | Component B [ppm by mass] | Moisture content [ppm by mass] | BMA dimer [ppm by area] | Purity of BMA [%] |
| Example A1 | 97.43 | 11 | 12 | 164 | 571 | 97.40 |
| Example A2 | 97.43 | 13 | 11 | 164 | 536 | 97.59 |
| Example A3 | 97.43 | 13 | 15 | 164 | 505 | 97.64 |
| Example A4 | 97.43 | 13 | 10 | 164 | 505 | 97.70 |
| Example A5 | 97.43 | 11 | 11 | 164 | 473 | 97.72 |
| Example A6 | 97.43 | 11 | 12 | 164 | 459 | 97.72 |
| Example A7 | 97.43 | 12 | 14 | 164 | 451 | 97.70 |
| Example A8 | 97.43 | 107 | 10 | 164 | 442 | 97.50 |
| Example A9 | 97.43 | 11 | 12 | 164 | 425 | 97.71 |
| Example A10 | 97.44 | 11 | 0 | 164 | 403 | 97.69 |
| Comparative Example A1 | 97.44 | 0 | 0 | 164 | 702 | 97.15 |

As shown in Table 2, in the ester-compound-containing compositions of Examples A1 to A10 in which the component A was contained, the generated amount of BMA dimer after the storage was smaller and the purity of BMA was higher than that of the ester-compound-containing composition of Comparative Example A1 in which the component A was not contained.

[Examples A11 to A13 and Comparative Examples A2 and A3]

An ester-compound-containing composition was prepared in the same manner as in Example A1, except that ethyl methacrylate (EMA, manufactured by Tokyo Chemical Industry Co., Ltd., moisture content: 245 ppm by mass) was

used instead of the BMA and the formulations of the A solution, the B solution, and the ester-compound-containing composition were changed as shown in Table 3, and the purity of EMA was obtained. The results are shown in Table 4.

[Table 3]

| | A solution | | | | B solution | | | | Ester-compound-containing composition | | |
|---|---|---|---|---|---|---|---|---|---|---|---|
| | EMA [g] | Component A | | | EMA [g] | Component B | | | EMA [g] | A solution [g] | B solution [g] |
| | | Compound name | Addition amount [g] | Contained amount [ppm by mass] | | Compound name | Addition amount [g] | Contained amount [ppm by mass] | | | |
| Example A11 | 10.0078 | Oct | 0.0248 | 2478 | 10.0166 | DMTBP | 0.0212 | 2116 | 5.0096 | 0.0245 | 0.0305 |
| Example A12 | 10.0067 | 2EH | 0.0209 | 2089 | 10.0166 | DMTBP | 0.0212 | 2116 | 5.0067 | 0.0255 | 0.0252 |
| Example A13 | 10.0101 | Ddc | 0.0206 | 2058 | 10.0166 | DMTBP | 0.0212 | 2116 | 5.0239 | 0.0252 | 0.0265 |
| Comparative Example A2 | - | - | - | - | 10.0166 | DMTBP | 0.0212 | 2116 | 5.0262 | - | 0.0263 |
| Comparative Example A3 | - | - | - | - | - | - | - | - | 5.0011 | - | - |

[Table 4]

| | Formulation of ester-compound-containing composition (calculated value) | | | | Analysis result of ester-compound-containing composition after storage |
|---|---|---|---|---|---|
| | EMA [% by mass] | Component A [ppm by mass] | Component B [ppm by mass] | Moisture content [ppm by mass] | Purity of EMA [%] |
| Example A11 | 96.86 | 12 | 13 | 245 | 96.76 |
| Example A12 | 96.86 | 11 | 11 | 245 | 96.78 |
| Example A13 | 96.86 | 10 | 11 | 245 | 96.76 |
| Comparative Example A2 | 96.86 | 0 | 11 | 245 | 96.27 |
| Comparative Example A3 | 96.86 | 0 | 0 | 245 | 96.70 |

As shown in Table 4, in the ester-compound-containing compositions of Examples A11 to A13 in which the component A was contained, the purity of EMA after the storage was higher than that of the ester-compound-containing compositions of Comparative Examples A2 and A3 in which the component A was not contained.

<Ester-compound-containing composition containing ester compound (1-2)>

[GC-MS measurement]

The analysis of the ester-compound-containing composition after storage was carried out using GC-MS, and a formulation was determined by the method described in the section of "Analysis of ester-compound-containing composition" above. QP-2010 Ultra manufactured by Shimadzu Corporation was used for the GC-MS measurement.

(GC conditions)

Column (product name: InertCap1, manufactured by GL Sciences Inc.)
length: 60 m, inner diameter: 0.25 mm, film thickness: 1.50 μm
Injection volume: 1.0 μL
Vaporization chamber temperature: 280°C
Column oven temperature: when analyzing a 2-hydroxymethacrylate-containing composition, held at 150°C for 1 minute, raised from 150°C to 260°C at 2.5 °C/min, and held at 260°C for 15 minutes; when analyzing an ethylene glycol dimethacrylate-containing composition, held at 200°C for 1 minute, raised from 200°C to 260°C at 2.0 °C/min, and held at 260°C for 29 minutes
Carrier gas: helium
Injection mode: split (split ratio: 25)
Control mode: constant linear velocity (35.0 cm/sec)
Pressure: 262.8 KPa
Total flow rate: 47.2 mL/min
Purge flow rate: 3.0 mL/min
Column flow rate: 1.70 mL/min

(MS conditions)

Ionization method: electron ionization (EI)
Ion source temperature: 200°C
Interface temperature: 300°C
m/z detection range: 30 to 600

Detection time: 60 minutes

[Moisture content]

A moisture content was quantified by the Karl Fischer method using a trace moisture content measuring device (product name: CA-200, manufactured by Nittoseiko Analytech Co., Ltd.).

[Example B1]

0.0224 g of 2-ethyl-1-hexene as a component A was added to 10.0028 g of 2-hydroxyethyl methacrylate (HEMA, manufactured by Tokyo Chemical Industry Co., Ltd., moisture content: 247 ppm by mass) to prepare an A solution.

In addition, 0.0218 g of 2,4-dimethyl-6-t-butylphenol as a component B was added to 10.0158 g of HEMA (moisture content: 247 ppm by mass) to prepare a B solution.

Next, 0.0304 g of the A solution and 0.0334 g of the B solution were added to 5.0098 g of HEMA (moisture content: 247 ppm by mass) to prepare an ester-compound-containing composition.

The obtained ester-compound-containing composition was stored at 25°C for 21 days, and a purity (%) of HEMA was calculated from a proportion (area%) of an area value of HEMA to the total area value of all components detected by the GC-MS measurement. In the calculation, a value obtained by subtracting the moisture content (% by mass) from 100% was multiplied by the area% of HEMA to calculate the purity of HEMA.

[Examples B2 to B5 and Comparative Examples B1 to B3]

An ester-compound-containing composition was prepared in the same manner as in Example B1, except that the formulations of the A solution, the B solution, and the ester-compound-containing composition were changed as shown in Table 5, and the purity of HEMA was obtained.

[Table 5]

| | A solution | | | | B solution | | | | Ester-compound-containing composition | | | |
|---|---|---|---|---|---|---|---|---|---|---|---|---|
| | HEMA [g] | Component A | | | HEMA [g] | Component B | | | HEMA [g] | A solution [g] | B solution [g] | Pure water [g] |
| | | Compound name | Addition amount [g] | Contained amount [ppm by mass] | | Compound name | Addition amount [g] | Contained amount [ppm by mass] | | | | |
| Example B1 | 10.0028 | 2EH | 0.0224 | 2239 | 10.0158 | DMTBP | 0.0218 | 2177 | 5.0098 | 0.0304 | 0.0334 | - |
| Example B2 | 10.0081 | Oct | 0.0237 | 2368 | 10.0144 | 4MP | 0.0198 | 1977 | 5.0090 | 0.0309 | 0.0337 | - |
| Example B3 | 10.0081 | Oct | 0.0237 | 2368 | 10.0009 | IPPPD | 0.0193 | 1930 | 5.0198 | 0.0319 | 0.0336 | - |
| Example B4 | 10.0081 | Oct | 0.0237 | 2368 | 10.0122 | IIO-TEMPO | 0.0355 | 3546 | 5.0258 | 0.0294 | 0.0315 | - |
| Example B5 | 10.0081 | Oct | 0.0237 | 2368 | - | - | - | - | 5.0033 | 0.0280 | - | - |
| Comparative Example B1 | - | - | - | - | 10.0158 | DMTBP | 0.0218 | 2177 | 5.0222 | - | 0.0294 | - |
| Comparative Example B2 | 12.0074 | Oct | 0.6057 | 50444 | 10.0158 | DMTBP | 0.0218 | 2177 | 2.5137 | 2.5176 | 0.0317 | - |
| Comparative Example B3 | 10.0081 | Oct | 0.0237 | 2368 | 10.0158 | DMTBP | 0.0218 | 2177 | 4.7512 | 0.0331 | 0.0272 | 0.169 |

Abbreviations in Table 5 have the following meanings.

HEMA: 2-hydroxyethyl methacrylate

Oct: 1-octene

2EH: 2-ethyl-1-hexene

DMTBP: 2,4-dimethyl-6-t-butylphenol

4MP: 4-methoxyphenol

IPPPD: N-isopropyl-N'-phenyl-p-phenylenediamine

HO-TEMPO: 4-hydroxy-2,2,6,6-tetramethylpiperidine-N-oxyl

Table 6 shows the measurement results of the purity of HEMA in each example.

[Table 6]

| | Formulation of ester-compound-containing composition (calculated value) | | | | Analysis result of ester-compound-containing composition after storage |
|---|---|---|---|---|---|
| | HEMA [% by mass] | Component A [ppm by mass] | Component B [ppm by mass] | Moisture content [ppm by mass] | Purity of HEMA [%] |
| Example B1 | 81.15 | 13 | 14 | 247 | 80.85 |
| Example B2 | 81.15 | 14 | 13 | 247 | 81.02 |
| Example B3 | 81.15 | 15 | 13 | 247 | 80.93 |
| Example B4 | 81.15 | 14 | 22 | 247 | 80.96 |
| Example B5 | 81.15 | 13 | - | 247 | 80.90 |
| Comparative Example B1 | 81.15 | - | 13 | 247 | 80.83 |
| Comparative Example B2 | 78.64 | 25083 | 14 | 247 | 73.14 |
| Comparative Example B3 | 77.75 | 16 | 12 | 34177 | 77.38 |

As shown in Table 6, in the ester-compound-containing compositions of Examples B1 to B5 in which the component A was contained, the purity of HEMA after the storage was higher than that of the ester-compound-containing compositions of Comparative Examples B1 to B3 in which the component A was not contained.

[Examples B6 and B7 and Comparative Examples B4 to B6]

An ester-compound-containing composition was prepared in the same manner as in Example B1, except that ethylene glycol dimethacrylate (EDMA, moisture content: 91 ppm by mass) was used instead of the HEMA and the formulations of the A solution, the B solution, and the ester-compound-containing composition were changed as shown in Table 7, and the purity of EDMA was obtained. The results are shown in Table 8.

[Table 7]

| | A solution | | | | B solution | | | | Ester-compound-containing composition | | | |
|---|---|---|---|---|---|---|---|---|---|---|---|---|
| | EDMA [g] | Component A | | | EDMA [g] | Component B | | | EDMA [g] | A solution [g] | B solution [g] | Pure water [g] |
| | | Compound name | Addition amount [g] | Contained amount [ppm by mass] | | Compound name | Addition amount [g] | Contained amount [ppm by mass] | | | | |
| Example B6 | 10.012 | Oct | 0.0247 | 2467 | 10.0088 | DMTBP | 0.0222 | 2218 | 5.0229 | 0.0315 | 0.0296 | - |
| Example B7 | 10.012 | Oct | 0.0247 | 2467 | - | - | - | - | 4.9923 | 0.0298 | - | - |
| Comparative Example B4 | - | - | - | - | 10.0088 | DMTBP | 0.0222 | 2218 | 5.0127 | - | 0.0316 | - |
| Comparative Example B5 | - | - | - | - | - | - | - | - | 5.0197 | - | - | - |
| Comparative Example B6 | 12.0432 | Oct | 0.6028 | 50053 | 10.0088 | DMTBP | 0.0222 | 2218 | 2.5111 | 2.5050 | 0.0320 | - |

[Table 8]

| | Formulation of ester-compound-containing composition (calculated value) | | | | Analysis result of ester-compound-containing composition after storage |
|---|---|---|---|---|---|
| | EDMA [% by mass] | Component A [ppm by mass] | Component B [ppm by mass] | Moisture content [ppm by mass] | Purity of EDMA [%] |
| Example B6 | 90.47 | 15 | 13 | 91 | 91.05 |
| Example B7 | 90.47 | 15 | - | 91 | 90.94 |
| Comparative Example B4 | 90.47 | - | 14 | 91 | 90.92 |
| Comparative Example B5 | 90.47 | - | - | 91 | 90.81 |
| Comparative Example B6 | 87.99 | 24838 | 14 | 91 | 77.84 |

As shown in Table 8, in the ester-compound-containing compositions of Examples B3 and B4 in which the component A was contained, the purity of EDMA after the storage was higher than that of the ester-compound-containing compositions of Comparative Examples B4 and B5 in which the component A was not contained or that the ester-compound-containing composition of Comparative Example B6 in which a large amount of the component A was contained.

<Ester-compound-containing composition containing ester compound (1-3)>

[GC-MS measurement]

The analysis of the ester-compound-containing composition after storage was carried out using GC-MS, and a formulation was determined by the method described in the section of "Analysis of ester-compound-containing composition" above. QP-2010 Ultra manufactured by Shimadzu Corporation was used for the GC-MS measurement.

(GC conditions)

Column (product name: InertCap1, manufactured by GL Sciences Inc.)
length: 60 m, inner diameter: 0.25 mm, film thickness: 1.50 μm
Injection volume: 1.0 μL
Vaporization chamber temperature: 280°C
Column oven temperature: when analyzing a glycidyl methacrylate-containing composition, held at 150°C for 1 minute, raised from 150°C to 260°C at 2.5 °C/min, and held at 260°C for 15 minutes; when analyzing a methoxyethyl methacrylate-containing composition, held at 60°C for 1 minute, raised from 60°C to 260°C at 5 °C/min, and held at 260°C for 9 minutes
Carrier gas: helium
Injection mode: split (split ratio: 25)
Control mode: constant linear velocity (35.0 cm/sec)
Pressure: 262.8 KPa
Total flow rate: 47.2 mL/min
Purge flow rate: 3.0 mL/min
Column flow rate: 1.70 mL/min

(MS conditions)

Ionization method: electron ionization (EI)
Ion source temperature: 200°C

Interface temperature: 300°C
m/z detection range: 30 to 600
Detection time: 50 minutes for the analysis of glycidyl methacrylate-containing composition, 60 minutes for the analysis of methoxyethyl methacrylate-containing composition

[Moisture content]

A moisture content was quantified by the Karl Fischer method using a trace moisture content measuring device (product name: CA-200, manufactured by Nittoseiko Analytech Co., Ltd.).

[Example C1]

0.0222 g of 1-octane as a component A was added to 10.0085 g of glycidyl methacrylate (GMA, manufactured by Tokyo Chemical Industry Co., Ltd., moisture content: 174 ppm by mass) to prepare an A solution.
In addition, 0.0214 g of 2,4-dimethyl-6-t-butylphenol as a component B was added to 10.0147 g of GMA (moisture content: 174 ppm by mass) to prepare a B solution.
Next, 0.0337 g of the A solution and 0.0309 g of the B solution were added to 5.0003 g of GMA (moisture content: 174 ppm by mass) to prepare an ester-compound-containing composition.
The obtained ester-compound-containing composition was stored at 25°C for 21 days, and a purity (%) of GMA was calculated from a proportion (area%) of an area value of GMA to the total area value of all components detected by the GC-MS measurement. In the calculation, a value obtained by subtracting the moisture content (% by mass) from 100% was multiplied by the area% of each component to calculate the purity of GMA.

[Examples C2 to C9 and Comparative Examples C1 to C3]

An ester-compound-containing composition was prepared in the same manner as in Example C1, except that the formulations of the A solution, the B solution, and the ester-compound-containing composition were changed as shown in Table 9, and the purity of GMA was obtained.

[Table 9]

| | A solution | | | | B solution | | | | Ester-compound-containing composition | | | |
|---|---|---|---|---|---|---|---|---|---|---|---|---|
| | GMA [g] | Component A | | | GMA [g] | Component B | | | GMA [g] | A solution [g] | B solution [g] | Pure water [g] |
| | | Compound name | Addition amount [g] | Contained amount [ppm by mass] | | Compound name | Addition amount [g] | Contained amount [ppm by mass] | | | | |
| Example C1 | 10.0085 | Oct | 0.0222 | 2218 | 10.0147 | DMTBP | 0.0214 | 2137 | 5.0003 | 0.0337 | 0.0309 | - |
| Example C2 | 10.0065 | 2EH | 0.0243 | 2428 | 10.0147 | DMTBP | 0.0214 | 2137 | 5.0037 | 0.0323 | 0.0307 | - |
| Example C3 | 10.0024 | Ddc | 0.0216 | 2159 | 10.0147 | DMTBP | 0.0214 | 2137 | 5.0059 | 0.0298 | 0.0263 | - |
| Example C4 | 10.0085 | Oct | 0.0222 | 2218 | 10.0090 | 4MP | 0.0201 | 2008 | 5.0022 | 0.0302 | 0.0309 | - |
| Example C5 | 10.0085 | Oct | 0.0222 | 2218 | 10.0008 | IPPPD | 0.0198 | 1980 | 5.0087 | 0.0308 | 0.0258 | - |
| Example C6 | 10.0085 | Oct | 0.0222 | 2218 | 10.0174 | PHT | 0.0313 | 3125 | 5.0045 | 0.0302 | 0.0308 | - |
| Example C7 | 10.0085 | Oct | 0.0222 | 2218 | 10.0105 | HO-TEMPO | 0.0206 | 2058 | 5.0020 | 0.0312 | 0.0289 | - |
| Example C8 | 10.0085 | Oct | 0.0222 | 2218 | 10.0147 | DMTBP | 0.0214 | 2137 | 4.7573 | 0.2592 | 0.0299 | - |
| Example C9 | 10.0085 | Oct | 0.0222 | 2218 | - | - | - | - | 5.0022 | 0.0291 | - | - |
| Comparative Example C1 | - | - | - | - | 10.0147 | DMTBP | 0.0214 | 2137 | 5.0028 | - | 0.0280 | - |
| Comparative Example C2 | - | - | - | - | - | - | - | - | 5.0004 | - | - | - |
| Comparative Example C3 | 10.0085 | Oct | 0.0222 | 2218 | 10.0147 | DMTBP | 0.0200 | 1997 | 4.7514 | 0.0337 | 0.0309 | 0.1484 |

Abbreviations in Table 9 have the following meanings.

GMA: glycidyl methacrylate

Oct: 1-octene

2EH: 2-ethyl-1-hexene

Ddc: 1-dodecene

DMTBP: 2,4-dimethyl-6-t-butylphenol

4MP: 4-methoxyphenol

IPPPD: N-isopropyl-N'-phenyl-p-phenylenediamine

PHT: phenothiazine

HO-TEMPO: 4-hydroxy-2,2,6,6-tetramethylpiperidine-N-oxyl

Table 10 shows the measurement results of the purity of GMA in each example.

[Table 10]

| | Formulation of ester-compound-containing composition (calculated value) | | | | Analysis result of ester-compound-containing composition after storage |
|---|---|---|---|---|---|
| | GMA [% by mass] | Component A [ppm by mass] | Component B [ppm by mass] | Moisture content [ppm by mass] | Purity of GMA [%] |
| Example C1 | 85.98 | 15 | 13 | 174 | 86.91 |
| Example C2 | 85.98 | 15 | 13 | 174 | 86.61 |
| Example C3 | 85.98 | 13 | 11 | 174 | 86.34 |
| Example C4 | 85.98 | 13 | 12 | 174 | 86.31 |
| Example C5 | 85.98 | 13 | 10 | 174 | 86.10 |
| Example C6 | 85.98 | 13 | 19 | 174 | 86.18 |
| Example C7 | 85.98 | 14 | 12 | 174 | 86.21 |
| Example C8 | 85.97 | 114 | 13 | 174 | 85.70 |
| Example C9 | 85.98 | 13 | | 174 | 85.77 |
| Comparative Example C1 | 85.98 | | 12 | 174 | 85.62 |
| Comparative Example C2 | 85.99 | | | 174 | 85.58 |
| Comparative Example C3 | 82.99 | 15 | 12 | 30067 | 82.71 |

As shown in Table 10, in the ester-compound-containing compositions of Examples C1 to C9 in which the component A was contained, the generated amounts of GMA dimer and glycidyl pyruvate after the storage were smaller and the purity of GMA was higher than those of the ester-compound-containing composition of Comparative Examples C1 and C2 in which the component A was not contained.

[Examples C10 to C12 and Comparative Examples C4 to C6]

An ester-compound-containing composition was prepared in the same manner as in Example C1, except that 2-methoxyethyl methacrylate (MTMA, manufactured by Tokyo Chemical Industry Co., Ltd., moisture content: 575 ppm by

mass) was used instead of the GMA and the formulations of the A solution, the B solution, and the ester-compound-containing composition were changed as shown in Table 11, and the generated amount of MTMA dimer and the purity of MTMA were obtained. The generated amount (% by mass) of MTMA dimer was obtained a proportion (area%) of an area value of ETMA dimer to the total area value of all detected components. The results are shown in Table 12.

[Table 11]

| | A solution | | | | B solution | | | | Ester-compound-containing composition | | | |
|---|---|---|---|---|---|---|---|---|---|---|---|---|
| | ETMA [g] | Component A | | | ETMA [g] | Component B | | | ETMA [g] | A solution [g] | B solution [g] | Pure water [g] |
| | | Compound name | Addition amount [g] | Contained amount [ppm by mass] | | Compound name | Addition amount [g] | Contained amount [ppm by mass] | | | | |
| Example C10 | 10.0271 | Oct | 0.0224 | 2234 | 10.0125 | DMTBP | 0.0259 | 2587 | 5.0004 | 0.0248 | 0.0298 | - |
| Example C11 | 10.0122 | 2EH | 0.0212 | 2117 | 10.0125 | DMTBP | 0.0259 | 2587 | 5.0142 | 0.0330 | 0.0276 | - |
| Example C12 | 10.0134 | Ddc | 0.0214 | 2137 | 10.0125 | DMTBP | 0.0259 | 2587 | 5.0080 | 0.0309 | 0.0278 | - |
| Comparative Example C4 | - | - | - | - | 10.0125 | DMTBP | 0.0259 | 2587 | 4.9993 | - | 0.0268 | - |
| Comparative Example C5 | - | - | - | - | - | - | - | - | 5.0138 | - | - | - |
| Comparative Example C6 | 10.0092 | Oct | 0.6019 | 60135 | 10.0125 | DMTBP | 0.0200 | 1998 | 2.5145 | 2.4990 | 0.0250 | - |

[Table 12]

| | Formulation of ester-compound-containing composition (calculated value) | | | | Analysis result of ester-compound-containing composition after storage | |
|---|---|---|---|---|---|---|
| | ETMA [% by mass] | Component A [ppm by mass] | Component B [ppm by mass] | Moisture content [ppm by mass] | ETMA dimer [% by area] | Purity of ETMA [%] |
| Example C10 | 99.21 | 11 | 15 | 575 | 0.23 | 99.39 |
| Example C11 | 99.21 | 14 | 14 | 575 | 0.23 | 99.40 |
| Example C12 | 99.21 | 13 | 14 | 575 | 0.23 | 99.39 |
| Comparative Example C4 | 99.21 | - | 14 | 575 | 0.31 | 99.25 |
| Comparative Example C5 | 99.22 | - | - | 575 | 0.30 | 99.27 |
| Comparative Example C6 | 96.23 | 29826 | 10 | 575 | 0.26 | 86.38 |

As shown in Table 12, in the ester-compound-containing compositions of Examples C10 to C12 in which the component A was contained, the generated amount of MTMA dimer after the storage was smaller and the purity of MTMA was higher than that of the ester-compound-containing composition of Comparative Examples C4 to C6 in which the component A was not contained.

[Example D1]

A mixture of butyl methacrylate (BMA, manufactured by Tokyo Chemical Industry Co., Ltd., moisture content: 164 ppm by mass) and ethyl methacrylate (EMA, manufactured by Tokyo Chemical Industry Co., Ltd., moisture content: 245 ppm by mass) was used as an ester compound, 1-octene was used as a component A, and 2,4-dimethyl-6-t-butylphenol was used as a component B. An ester-compound-containing composition was prepared in the same manner as in Example A1, except that the formulation of the ester-compound-containing composition was changed as shown in Table 13, and the generated amount of BMA dimer, the purity of BMA, and the purity of EMA were obtained. The results are shown in Table 13.

The purity (%) of BMA was calculated from a proportion (area%) of an area value of BMA to the total area value of all detected components, and the purity (%) of EMA was calculated from a proportion (area%) of an area value of EMA to the total area value of all detected components. In the calculation, a value obtained by subtracting the moisture content (% by mass) from 100% was multiplied by the area% of each component to calculate the BMA dimer, the purity of BMA, and the purity of EMA.

[Comparative Examples D1 and D2]

An ester-compound-containing composition was prepared in the same manner as in Example D1, except that the formulation of the ester-compound-containing composition was changed as shown in Table 13, and the generated amount of BMA dimer, the purity of BMA, and the purity of EMA were obtained. The results are shown in Table 13.

[Table 13]

| | Formulation of ester-compound-containing composition (calculated value) | | | | | Analysis result of ester-compound-containing composition after storage | | |
|---|---|---|---|---|---|---|---|---|
| | BMA [% by mass] | EMA [% by mass] | Component A [ppm by mass] | Component B [ppm by mass] | Moisture content [ppm by mass] | BMA dimer [ppm by area] | Purity of BMA [%] | Purity of EMA [%] |
| Example D1 | 82.82 | 14.61 | 18 | 21 | 176 | 299 | 77.73 | 20.21 |
| Comparative Example D1 | 82.82 | 14.61 | 0 | 20 | 176 | 450 | 77.56 | 20.46 |

(continued)

| | Formulation of ester-compound-containing composition (calculated value) | | | | | Analysis result of ester-compound-containing composition after storage | | |
|---|---|---|---|---|---|---|---|---|
| | BMA [% by mass] | EMA [% by mass] | Component A [ppm by mass] | Component B [ppm by mass] | Moisture content [ppm by mass] | BMA dimer [ppm by area] | Purity of BMA [%] | Purity of EMA [%] |
| Comparative Example D2 | 82.82 | 14.61 | 0 | 0 | 176 | 453 | 77.52 | 20.38 |

As shown in Table 13, in the ester-compound-containing composition of Example D1 in which the component A was contained, the generated amount of BMA dimer after the storage was smaller and the purity of BMA was higher than that of the ester-compound-containing compositions of Comparative Examples D1 and D2 in which the component A was not contained.

[Example D2]

Butyl methacrylate (BMA, manufactured by Tokyo Chemical Industry Co., Ltd., moisture content: 164 ppm by mass) was used as an ester compound, 1-octene was used as a component A, and 2,4-dimethyl-6-t-butylphenol was used as a component B. An ester-compound-containing composition was prepared in the same manner as in Example A1, except that the formulation of the ester-compound-containing composition was changed as shown in Table 14, and the generated amount of BMA dimer and the purity of BMA were obtained. The results are shown in Table 14.

[Examples D3 and D4]

An ester-compound-containing composition was prepared in the same manner as in Example D2, except that methanol (manufactured by NACALAI TESQUE, INC., for high-speed liquid chromatography) was added to change the formulation of the ester-compound-containing composition as shown in Table 14, and the generated amount of BMA dimer and the purity of BMA were obtained. The results are shown in Table 14.

[Table 14]

| | Formulation of ester-compound-containing composition (calculated value) | | | | Analysis result of ester-compound-containing composition after storage | |
|---|---|---|---|---|---|---|
| | BMA [% by mass] | Component A [ppm by mass] | Component B [ppm by mass] | Moisture content [ppm by mass] | BMA dimer [ppm by area] | Purity of BMA [%] |
| Example D2 | 97.30 | 17 | 20 | 1541 | 370 | 97.58 |
| Example D3 | 82.82 | 17 | 20 | 241 | 549 | 77.73 |
| Example D4 | 82.82 | 17 | 0 | 241 | 525 | 77.60 |

As shown in Table 14, when comparing the ester-compound-containing compositions of Examples D3 and D4 in which the component A was contained and the concentration of BMA was similar, the generated amount of BMA dimer was similar in Example D3 in which the component A and the component B were used in combination and Example D4 in which only the component A was used. On the other hand, the ester-compound-containing composition of Example D2 in which the component A was contained and the concentration of BMA was high had a smaller generated amount of BMA than the ester-compound-containing composition of Example D3 in which the component A was contained and the concentration of BMA was low.

[Example D5]

Butyl methacrylate (BMA, manufactured by Tokyo Chemical Industry Co., Ltd., moisture content: 164 ppm by

mass) was used as an ester compound, 1-octene was used as a component A, and 2,4-dimethyl-6-t-butylphenol was used as a component B. An ester-compound-containing composition was prepared in the same manner as in Example A1, except that the formulation of the ester-compound-containing composition was changed as shown in Table 15, and the generated amount of BMA dimer and the purity of BMA were obtained. The results are shown in Table 15.

[Example D6 and Comparative Example D3]

An ester-compound-containing composition was prepared in the same manner as in Example D2, except that the formulation of the ester-compound-containing composition was changed as shown in Table 15, and the generated amount of BMA dimer and the purity of BMA were obtained. The results are shown in Table 15.

[Table 15]

| | Formulation of ester-compound-containing composition (calculated value) | | | | Analysis result of ester-compound-containing composition after storage | |
|---|---|---|---|---|---|---|
| | BMA [% by mass] | Component A [ppm by mass] | Component B [ppm by mass] | Moisture content [ppm by mass] | BMA dimer [ppm by area] | Purity of BMA [%] |
| Example D5 | 97.42 | 131 | 20 | 176 | 384 | 97.58 |
| Example D6 | 97.34 | 920 | 19 | 176 | 437 | 97.44 |
| Comparative Example D3 | 97.25 | 1870 | 18 | 176 | 632 | 97.26 |

As shown in Table 15, in the ester-compound-containing compositions of Examples D5 and D6 in which the component A was contained, the generated amount of BMA dimer after the storage was smaller and the purity of BMA was higher than that of the ester-compound-containing composition of Comparative Example D3 in which the concentration of the component A was too large.

[Example D7]

Butyl methacrylate (BMA, manufactured by Tokyo Chemical Industry Co., Ltd., moisture content: 164 ppm by mass) was used as an ester compound, 1-octene was used as a component A, and 2,4-dimethyl-6-t-butylphenol was used as a component B. An ester-compound-containing composition was prepared in the same manner as in Example A1, except that the formulation of the ester-compound-containing composition was changed as shown in Table 16, and the generated amount of BMA dimer was obtained. The results are shown in Table 16.

[Example D8 and Comparative Example D4]

An ester-compound-containing composition was prepared in the same manner as in Example D2, except that the formulation of the ester-compound-containing composition was changed as shown in Table 16, and the generated amount of BMA dimer was obtained. The results are shown in Table 16.

[Table 16]

| | Formulation of ester-compound-containing composition (calculated value) | | | | Analysis result of ester-compound-containing composition after storage |
|---|---|---|---|---|---|
| | BMA [% by mass] | Component A [ppm by mass] | Component B [ppm by mass] | Moisture content [ppm by mass] | BMA dimer [ppm by area] |
| Example D7 | 97.12 | 17 | 19 | 3290 | 387 |
| Example D8 | 96.99 | 17 | 20 | 4648 | 396 |
| Comparative Example D4 | 96.39 | 17 | 19 | 10588 | 553 |

As shown in Table 16, in the ester-compound-containing compositions of Examples D7 and D8 in which the component A was contained and the moisture content was a specified amount or less, the generated amount of BMA dimer after the storage was smaller than that of the ester-compound-containing composition of Comparative Example D4 in which the moisture content was too large.

INDUSTRIAL APPLICABILITY

According to the present invention, an ester-compound-containing composition which can be used as a raw material or the like of a (meth)acrylic polymer can be stably stored for a long period of time, which is industrially useful.

## Claims

**1.** An ester-compound-containing composition, comprising:

an ester compound (1) represented by Formula (1); and
a component A: an $\alpha$-olefin,
wherein a contained amount of the component A is 1 ppm by mass or more and 1500 ppm by mass or less, and a moisture content is 5000 ppm by mass or less,

$$CH_2=CR^1\text{-}C(=O)\text{-}O\text{-}R^2 \cdots \qquad (1),$$

wherein, in Formula (1), $R^1$ is a hydrogen atom or a methyl group, and $R^2$ is a hydrocarbon group having 2 to 20 carbon atoms, which may have a heteroatom and may have a functional group.

**2.** The ester-compound-containing composition according to Claim 1, wherein $R^2$ in Formula (1) is a hydrocarbon group having 2 to 20 carbon atoms, a linear or branched alkyl group having 2 to 8 carbon atoms, in which at least one hydrogen atom is substituted with a hydroxy group, a group in which one or two hydrogen atoms in a linear or branched alkyl group or hydroxyalkyl group having 2 to 8 carbon atoms are substituted with a (meth)acryloyloxy group, or a group having an etheric oxygen between carbon atoms of a hydrocarbon group having 2 to 8 carbon atoms.

**3.** The ester-compound-containing composition according to Claim 1,

wherein the ester compound (1) includes an ester compound (1-1) represented by Formula (1-1),

$$CH_2=CR^{a1}\text{-}C(=O)\text{-}O\text{-}R^{a2} \cdots \qquad (1\text{-}1),$$

(here, in Formula (1-1), $R^{a1}$ is a hydrogen atom or a methyl group, and $R^{a2}$ is a hydrocarbon group having 2 to 20 carbon atoms)

**4.** The ester-compound-containing composition according to Claim 3, wherein the ester compound (1-1) in which $R^{a2}$ is a linear or branched alkyl group having 2 to 20 carbon atoms is included.

**5.** The ester-compound-containing composition according to Claim 3, wherein a contained amount of the ester compound (1-1) is 85.00% by mass or more and 99.99% by mass or less.

**6.** The ester-compound-containing composition according to Claim 1,

wherein the ester compound (1) includes one or more ester compounds (1-2) selected from the group consisting of an ester compound (1-21) represented by Formula (1-21), an ester compound (1-22) represented by Formula (1-22), and an ester compound (1-23) represented by Formula (1-23),

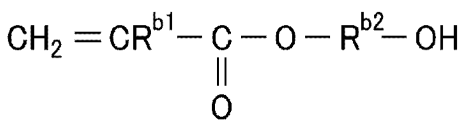 ・・・(1－21)

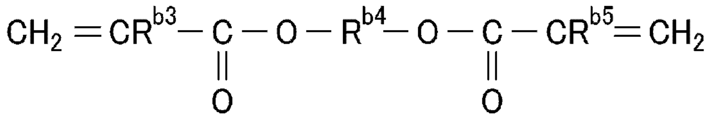

・・・(1−22)

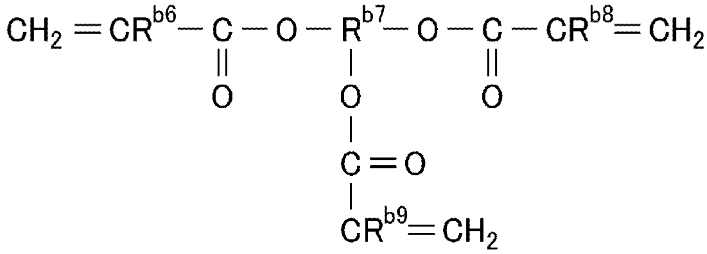

・・・(1−23)

wherein, in Formulae (1-21) to (1-23), $R^{b1}$, $R^{b3}$, $R^{b5}$, $R^{b6}$, $R^{b8}$, and $R^{b9}$ are each independently a hydrogen atom or a methyl group, $R^{b2}$ and $R^{b4}$ are each independently a linear or branched alkylene group or hydroxyalkylene group having 2 to 8 carbon atoms, and $R^{b7}$ is a linear or branched trivalent hydrocarbon group having 2 to 8 carbon atoms.

**7.** The ester-compound-containing composition according to Claim 6,
wherein the ester compound (1-2) includes one or more selected from the group consisting of ethylene glycol di(meth) acrylate, 2-hydroxyethyl (meth)acrylate, 3-hydroxypropyl (meth)acrylate, and trimethylolpropane tri(meth)acrylate.

**8.** The ester-compound-containing composition according to Claim 6,
wherein a contained amount of the ester compound (1-2) is 85.00% by mass or more and 99.99% by mass or less.

**9.** The ester-compound-containing composition according to Claim 1,

wherein the ester compound (1) includes an ester compound (1-3) represented by Formula (1-3),

$CH_2=CR^{c1}-C(=O)-O-R^{c2}$ ··· (1-3),

wherein, in Formula (1-3), $R^{c1}$ is a hydrogen atom or a methyl group, and $R^{c2}$ is a group having an etheric oxygen between carbon atoms of a hydrocarbon group having 2 to 8 carbon atoms.

**10.** The ester-compound-containing composition according to Claim 9,
wherein the ester compound (1-3) in which $R^{c2}$ is a 2-methoxyethyl group, a glycidyl group, or a tetrahydrofurfuryl group is included.

**11.** The ester-compound-containing composition according to Claim 9,
wherein a contained amount of the ester compound (1-3) is 85.00% by mass or more and 99.99% by mass or less.

**12.** The ester-compound-containing composition according to Claim 1,
wherein the component A includes an $\alpha$-olefin having 6 or more and 12 or less carbon atoms.

**13.** The ester-compound-containing composition according to Claim 1, further comprising:
a component B: a polymerization inhibitor.

**14.** The ester-compound-containing composition according to Claim 1,
wherein the ester-compound-containing composition is stored for 1 day or longer.

**15.** A method for producing an ester-compound-containing composition containing an ester compound (1) represented by Formula (1), the production method comprising:

performing an ester exchange reaction between methyl (meth)acrylate and an alcohol (a) having 2 to 20 carbon atoms in a presence of a component A: an $\alpha$-olefin,

$CH_2=CR^1-C(=O)-O-R^2$ ··· (1),

wherein, in Formula (1), $R^1$ is a hydrogen atom or a methyl group, and $R^2$ is a hydrocarbon group having 2 to 20 carbon atoms, which may have a heteroatom and may have a functional group.

**16.** The method for producing an ester-compound-containing composition according to Claim 15,

wherein $R^2$ in Formula (1) is a hydrocarbon group having 2 to 20 carbon atoms, a linear or branched alkyl group having 2 to 8 carbon atoms, in which at least one hydrogen atom is substituted with a hydroxy group, a group in which one or two hydrogen atoms in a linear or branched alkyl group or hydroxyalkyl group having 2 to 8 carbon atoms are substituted with a (meth)acryloyloxy group, or a group having an etheric oxygen between carbon atoms of a hydrocarbon group having 2 to 8 carbon atoms, and
the alcohol (a) is at least one selected from the following alcohols (a1) to (a3),
(a1) a monoalcohol having 2 to 20 carbon atoms,
(a2) one or more alcohols selected from the group consisting of a dialcohol having 2 to 8 carbon atoms and a trialcohol having 2 to 8 carbon atoms,
(a3) an ether-bond-containing alcohol having 2 to 8 carbon atoms.

**17.** The method for producing an ester-compound-containing composition according to Claim 16,

wherein an ester compound (1-1) represented by Formula (1-1) is obtained by the ester exchange reaction between the methyl (meth)acrylate and the alcohol (a1) in the presence of the component A,

$$CH_2=CR^{a1}\text{-}C(=O)\text{-}O\text{-}R^{a2} \cdots \qquad (1\text{-}1),$$

wherein, in Formula (1-1), $R^{a1}$ is a hydrogen atom or a methyl group, and $R^{a2}$ is a hydrocarbon group having 2 to 20 carbon atoms.

**18.** The method for producing an ester-compound-containing composition according to Claim 17, wherein the alcohol (a1) includes a linear or branched monoalcohol having 2 to 20 carbon atoms.

**19.** The method for producing an ester-compound-containing composition according to Claim 16,

wherein an ester compound (1-2) including one or more ester compounds (1-2) selected from the group consisting of an ester compound (1-21) represented by Formula (1-21), an ester compound (1-22) represented by Formula (1-22), and an ester compound (1-23) represented by Formula (1-23) is obtained by the ester exchange reaction between the methyl (meth)acrylate and the alcohol (a2) in the presence of the component A,

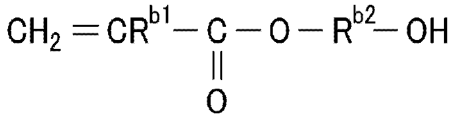
・・・(1−21)

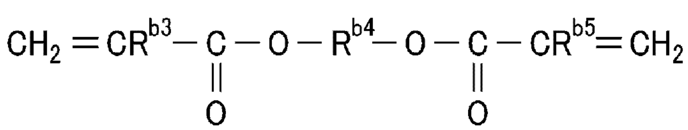
・・・(1−22)

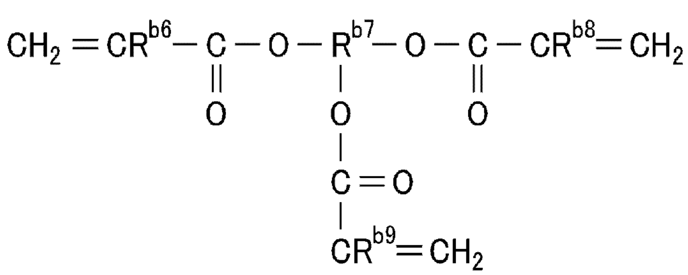
・・・(1−23)

wherein, in Formulae (2-1) to (2-3), $R^{b1}$, $R^{b3}$, $R^{b5}$, $R^{b6}$, $R^{b8}$, and $R^{b9}$ are each independently a hydrogen atom or a

methyl group, $R^{b2}$ and $R^{b4}$ are each independently a linear or branched alkylene group or hydroxyalkylene group having 2 to 8 carbon atoms, and $R^{b7}$ is a linear or branched trivalent hydrocarbon group having 2 to 8 carbon atoms.

**20.** The method for producing an ester-compound-containing composition according to Claim 19, wherein the alcohol (a2) includes one or more selected from the group consisting of ethylene glycol, 1,2-propanediol, 1,3-propanediol, and trimethylolpropane.

**21.** The method for producing an ester-compound-containing composition according to Claim 16,

wherein an ester compound (1-3) represented by Formula (1-3) is obtained by the ester exchange reaction between the methyl (meth)acrylate and the alcohol (a3) in the presence of the component A,

$$CH_2=CR^{c1}-C(=O)-O-R^{c2} \cdots \qquad (1-3),$$

wherein, in Formula (1-3), $R^{c1}$ is a hydrogen atom or a methyl group, and $R^{c2}$ is a group having an etheric oxygen between carbon atoms of a hydrocarbon group having 2 to 8 carbon atoms.

**22.** The method for producing an ester-compound-containing composition according to Claim 21, wherein the alcohol (a3) includes one or more selected from the group consisting of 2-methoxyethanol, glycidyl alcohol, and tetrahydrofurfuryl alcohol.

**23.** The method for producing an ester-compound-containing composition according to any one of Claims 15 to 22, wherein the component A includes an $\alpha$-olefin having 6 or more and 12 or less carbon atoms.

**24.** A polymerizable composition for producing a (meth)acrylic polymer, comprising: the ester-compound-containing composition according to Claim 1.

**25.** The polymerizable composition according to Claim 24, wherein a contained amount of a component C: at least one compound selected from the group consisting of a compound of a transition metal and a compound of Group 13 element is $7 \times 10^4$ ppm by mass or less with respect to a total mass of the component A in the ester-compound-containing composition.

**26.** A method for producing a (meth)acrylic polymer, comprising: polymerizing the polymerizable composition according to Claim 24.

**27.** A method for producing a (meth)acrylic polymer, comprising: polymerizing the polymerizable composition according to Claim 25.

## INTERNATIONAL SEARCH REPORT

International application No.
**PCT/JP2023/034093**

### A. CLASSIFICATION OF SUBJECT MATTER

***C08F 20/18***(2006.01)i; ***C07C 67/03***(2006.01)i; ***C07C 67/62***(2006.01)i; ***C07C 69/54***(2006.01)i; ***C07D 303/16***(2006.01)i; ***C07D 307/12***(2006.01)i; ***C08F 10/14***(2006.01)i; ***C08F 20/20***(2006.01)i; ***C08F 20/28***(2006.01)i
FI: C08F20/18; C07C67/03; C07C67/62; C07C69/54 Z; C07D303/16; C07D307/12; C08F10/14; C08F20/20; C08F20/28

According to International Patent Classification (IPC) or to both national classification and IPC

### B. FIELDS SEARCHED

Minimum documentation searched (classification system followed by classification symbols)

C08F20/18; C07C67/03; C07C67/62; C07C69/54; C07D303/16; C07D307/12; C08F10/14; C08F20/20; C08F20/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| A | JP 2012-512859 A (LUCITE INTERNATIONAL UK LTD.) 07 June 2012 (2012-06-07) | 1-27 |
| A | JP 2009-62289 A (NIPPON SHOKUBAI CO., LTD.) 26 March 2009 (2009-03-26) | 1-27 |
| A | JP 2009-24010 A (BASF SE) 05 February 2009 (2009-02-05) | 1-27 |
| A | JP 2004-217792 A (MITSUBISHI RAYON CO., LTD.) 05 August 2004 (2004-08-05) | 1-27 |
| A | JP 2020-158686 A (KURARAY CO., LTD.) 01 October 2020 (2020-10-01) | 1-27 |
| P, X | WO 2022/196522 A1 (MITSUBISHI CHEMICAL CORP.) 22 September 2022 (2022-09-22) | 1-27 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 November 2023** | **28 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/034093**

| Patent document cited in search report | Publication date (day/month/year) | Patent family member(s) | Publication date (day/month/year) |
|---|---|---|---|
| JP 2012-512859 A | 07 June 2012 | US 2011/0282023 A1 | |
| | | WO 2010/070325 A1 | |
| | | EP 2370393 A1 | |
| | | CA 2744402 A1 | |
| | | TW 201035041 A | |
| | | CN 102256926 A | |
| | | KR 10-2011-0101209 A | |
| JP 2009-62289 A | 26 March 2009 | (Family: none) | |
| JP 2009-24010 A | 05 February 2009 | JP 2013-147501 A | |
| | | JP 2015-155481 A | |
| | | US 2009/0023947 A1 | |
| | | EP 2017255 A1 | |
| | | DE 102008002923 A | |
| JP 2004-217792 A | 05 August 2004 | (Family: none) | |
| JP 2020-158686 A | 01 October 2020 | (Family: none) | |
| WO 2022/196522 A1 | 22 September 2022 | JP 2023-11905 A | |
| | | TW 202239769 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022149219 A
- JP 2022149220 A
- JP 2022149221 A
- JP 2009274986 A


### Non-patent literature cited in the description


- **ITTEL et al.** Late-Metal Catalysts for Ethylene Homo- and Copolymerization. *Chemical Reviews*, 25 March 2000, vol. 100 (4), 1169-1204